# EUROPEAN PATENT APPLICATION

(11) **EP 0 622 459 A1**
(43) Date of publication of application: **02.11.1994**
(21) Application number: 94106392.7
(22) Date of filing: 25.04.1994
(51) Int. Cl.: C12N 15/62, C12N 15/12, C07K 7/10, C12N 1/21

(54) **Fused proteins for preparing vasoactive intestinal polypeptide analogs, method of preparing same and recombinant plasmids and transformant microorganisms**

(30) Priority: 26.04.1993 JP 99313/93
(71) Applicant: SANWA KAGAKU KENKYUSHO CO., LTD., Higashi-ku Nagoya-shi Aichi-ken (JP)
(72) Inventor: Takahashi, Haruo, c/o Sanwa Kagaku Kenkyusho CoLtd, Nagoya-shi, Aichi (JP); Kobayashi, Yohei, c/o Sanwa Kagaku Kenkyusho CoLtd, Nagoya-shi, Aichi (JP); Nakoshi, Masanao, c/o Sanwa Kagaku Kenkyusho CoLtd, Nagoya-shi, Aichi (JP); Mitani, Takahiko, c/o Sanwa Kagaku Kenkyusho CoLtd, Nagoya-shi, Aichi (JP); Hirade, Kinya, c/o Sanwa Kagaku Kenkyusho CoLtd, Nagoya-shi, Aichi (JP); Sawai, Kiichi, c/o Sanwa Kagaku Kenkyusho CoLtd, Nagoya-shi, Aichi (JP)
(74) Representative: Weisert, Annekäte, Dipl.-Ing. Dr.-Ing.

(57) **Abstract**

The invention provides a process for preparing, in large quantities, VIP analogs having potent biological activity and good stability by utilizing recombinant DNA technology.

Thus, a DNA coding for a peptide comprising a leader peptide derived from Sarcophaga lectin and one molecule, or a plurality of molecules arranged in tandem, of a polypeptide of the general formula: 
His-Ser-Asp-Ala-Val-Phe-Thr-R1-R2-Tyr-Thr-R3-Leu-Arg-Lys-Gln-
Leu-Ala-R4-R5-Lys-Tyr-Leu-R6-R7-R8-R9-R10-Met
wherein R₁ is Asp or Gly, R₂ is Asn or Gln, R₃ is Arg or Lys, R₄ is Val, Ala or Lys, R₅ is Lys or Leu, R₆ is Asn, Gln or Lys, R₇ is Ser, Lys or Ala, R₈ is Ile, Ala or Leu, R₉ is Leu or Lys and R₁₀ is Asn, Lys or Arg, is inserted into an expression plasmid, Escherichia coli is transformed with the resulting recombinant plasmid, and the transformant is cultured to give large quantity expression of the above-mentioned polypeptide in the form of a fused protein. Treatment of the fused protein with cyanogen bromide gives a biologically active VIP analog (in which the C-terminal amino acid residue is not Met but a homoserine or homoserine lactone residue derived from Met).

## Description

### FIELD OF THE INVENTION

This invention relates to fused proteins for preparing vasoactive intestinal polypeptide (VIP) analogs, to a method of producing VIP analogs, and to recombinant plasmids and transformant microorganisms for producing the fused proteins.

### BACKGROUND OF THE INVENTION

A number of smooth muscle relaxant polypeptides have been discovered. VIP is a peptide hormone isolated and purified in 1970 by Said and Mutt from a subfraction in the process of secretin extraction and purification from the porcine duodenum. It consists of 28 amino acid residues with a C-terminal amide. The primary structure (amino acid sequence) of VIP was elucidated in 1974 and found to be similar to that of glucagon or secretin, for instance, and, therefore, VIP is now regarded as a peptide hormone belonging to the glucagon-secretin family.

Later, VIP was found to be present not only in the digestive tract but also in the nervous system and so forth. It has diverse biological activities. Thus, reportedly, it has potent vasodilating and hypotensive activity, smooth muscle relaxant activity, intestinal juice secretion promoting activity, pancreatic juice and bile secretion promoting activity, gastric juice secretion suppressing activity, glycogenolytic activity, pituitary hormone secretion promoting activity, genitalial blood flow regulating activity and bronchodilating activity, among others.

Among these biological activities, the bronchial smooth muscle relaxant activity of VIP is expected to be useful in the treatment of bronchial asthma and the vasodilating activity of VIP on trabeculae corporum cavernosorum of the penis is expected to be useful in the treatment of impotence.

However, extraction and isolation of VIP from animal organs or chemical synthesis of VIP has many problems, especially with respect to productivity, production efficiency, purity and production cost. Furthermore, VIP is physico-chemically unstable and can be readily decomposed by enzymes found in the living body. Therefore, VIP has been considered very difficult to put into practical use as a drug.

To solve the above problems, earnest studies have been made on VIP analogs at least comparable in biological activity to VIP or VIP analogs superior in stability to VIP. Thus, for example, the following study results have been reported.

As regards chemically synthesized VIP analogs, polypeptides resulting from substitution of a leucine or norleucine residue for the methionine residue at position 17 of VIP have similar biological activities to those of VIP (JP-A-62-246595, the term "JP-A" as used herein means an "unexamined published Japanese Patent Application" or European Patent Application EP 241,926). Deletion of one or two N-terminal or C-terminal amino acid residues from VIP results in a marked decrease in biological activity [J. Biol. Chem., vol. 266, pages 6389-6392 (1991)] whereas VIP analogs derived by addition of a basic amino acid residue to the C terminus are 3- to 4-fold more potent in biological activity (JP-A-62-246595, already cited above). VIP analogs resulting from acetylation at the N-terminus and substitution of a norleucine residue for the methionine residue at position 17 and further replacing several amino acid residues with respectively different amino acid residues show excellent biological activities (JP-A-3-38600 or European Patent Application EP 405,242).

However, these chemically synthesized VIP analogs either contain, in their structure, an amino acid residue not occurring in nature or are N-terminally acetylated. Therefore, it is practically impossible to produce them by utilizing recombinant DNA technology.

As regards genetically engineered VIP analogs, [Leu¹⁷]-VIP-OH (JP-A-1-296996 and JP-A-3-280893), [Leu¹⁷]-VIP-Gly, [Leu¹⁷]-VIP-Gly-Lys-Arg and the like (JP-A-3-502880) and VIP-Gly (JP-A-4-148694 or International Patent Application WO 92/06211) have been reported.

Attempts to prepare VIP using the processes disclosed in the patent specifications just cited above generally result in the expression of only several hundred micrograms, at most several milligrams, per liter of culture medium. For enabling commercial production thereof, it is therefore necessary to further increase the expression thereof.

When the VIP analogs disclosed in the above patent specifications are evaluated from the viewpoint of biological activity, Leu¹⁷-VIP-OH and VIP-Gly are only comparable to naturally occurring VIP and Leu¹⁷-VIP-Gly is about 70% active as compared with naturally occurring VIP; any increase in biological activity cannot be observed at all. The known genetically engineered VIP analogs thus have drawbacks from both the production efficiency and biological activity viewpoints.

To overcome such problems in the prior art, the present inventors made intensive investigations, found VIP analogs suited for large-scale commercial production utilizing the recombinant DNA technology and markedly improved in biological activity, and filed patent applications relative to the analogs (JP-A-4-230400 or European Patent Application EP 463,450, and U.S. Patent Application Serial No. 08/183,847 filed on January 21, 1994). The structural feature common to the VIP analogs disclosed by the present inventors in these patent applications is the addition of Hse (homoserine or a homoserine lactone residue) at the C terminus. These VIP analogs can be produced by mass expression, by the recombinant DNA technology, of a fused protein in which a plurality of molecules of a VIP analog precursor (with Met at the C terminus) are connected in series, followed by treatment of the fused protein with cyanogen bromide. (Cyanogen bromide treatment causes cleavage at the Met, dividing the fused protein into individual peptide fragments, with the simultaneous conversion of the C-terminal Met of each fragment to the above-mentioned Hse. Whether a homoserine residue or a homoserine lactone residue (or a mixture of these) is formed depends on the pH conditions.)
It is difficult to produce VIP and the known VIP analogs on a large commercial scale by conventional technology. Furthermore, VIP and the known VIP analogs are unstable in vivo and exhibit activity only for a short time. Therefore, it is necessary to administer them to living organisms at relatively high doses.

Accordingly, the primary object of the invention is to provide VIP analogs that can be produced on a large scale by utilizing the per se known recombinant DNA technology and that show potent in vivo biological activities, thereby being effective at reduced doses. This opens the way for practical use of VIP analog-containing pharmaceutical preparations as bronchodilators or therapeutic agents for impotence, among others.

A specific object of the invention is to provide fused proteins for preparing VIP analogs having potent biological activities.

Another specific object of the invention is to provide a method of preparing VIP analogs from said fused proteins.

A further specific object of the invention is to provide recombinant plasmids for expression of such VIP analogs, and microorganisms transformed with said plasmids.

To solve the problems mentioned above and achieve the above objects, the present inventors made further investigations and, as a result, found that [Leu¹⁷]-VIP-Hse (inclusive of Hse lactone) having potent biological activities can be produced in large quantities by inserting, into an expression plasmid, an appropriate leader peptide and a recombinant DNA corresponding to a peptide resulting from connection of [Leu¹⁷)-VIP-Met, preferably a plurality of [Leu¹⁷]-VIP-Met molecules arranged in tandem, linked to the leader peptide in a downstream direction, introducing the resulting recombinant plasmid into a host microorganism, culturing the transformant microorganism for the expression of a fused protein and its formation as an inclusion body and subjecting the fused protein to cyanogen bromide treatment to thereby divide the same into individual VIP analog molecules. The present invention has been completed based on this finding.

### SUMMARY OF THE INVENTION

The first specific object is accomplished by providing a fused protein for VIP analog production which comprises a leader peptide derived from Sarcophaga lectin, and one molecule, or a plurality of molecules arranged in tandem, of a peptide of the general formula (1): 
His-Ser-Asp-Ala-Val-Phe-Thr-R1-R2-Tyr-Thr-R3-Leu-Arg-Lys-Gln-
Leu-Ala-R4-R5-Lys-Tyr-Leu-R6-R7-R8-R9-R10-Met (SEQ ID NO: 1)
wherein R₁ is Asp or Gly, R₂ is Asn or Gln, R₃ is Arg or Lys, R₄ is Val, Ala or Lys, R₅ is Lys or Leu, R₆ is Asn, Gln or Lys, R₇ is Ser, Lys or Ala, R₈ is Ile, Ala or Leu, R₉ is Leu or Lys and R₁₀ is Asn, Lys or Arg, linked to the leader. peptide in a downstream direction.

The peptide of general formula (1) (SEQ ID NO: 1) is linked to the leader peptide derived from Sarcophaga lectin to stabilize the fused protein as an inclusion body. Although there is no particular limit to the molecular weight of the leader peptide, the leader peptide having a molecular weight of 5,000 or more is preferable, because the fused protein tends to not be expressed when the molecular weight is too lower. On the other hand, the upper limit of the molecular weight is preferably 40,000 or less. An excessively high molecular weight is undesirable since the relative proportion of the leader peptide in the fused protein formed becomes large (the leader peptide region is eventually discarded).

Preferred among the peptides of the above general formula (1) (SEQ ID NO: 1) are those in which R₁ is Gly, R₂ is Asn, R₃ is Lys, R₄ is Ala, R₅ is Lys, R₆ is Asn or Lys, R₇ is Lys or Ala, R₈ is Ala or Leu, R₉ is Leu or Lys and R₁₀ is Lys or Arg. The most preferred is 
His-Ser-Asp-Ala-Val-Phe-
Thr-Asp-Asn-Tyr-Thr-Arg-Leu-Arg-Lys-Gln-Leu-Ala-Val-Lys-Lys-
Tyr-Leu-Asn-Ser-Ile-Leu-Asn-Met (SEQ ID NO:3),
namely the [Leu¹⁷]-VIP-Met molecule.

The number of molecules of general formula (1) (SEQ ID NO: 1) to be linked in tandem is not critical. When the labor, reagents, time and other factors required for the linking are taken into consideration, however, the recommended number is about 1 to 15. The proportion of the fused protein to the total cell protein is highest when four to ten molecules are linked. Accordingly, this embodiment is preferred.

VIP analogs of the general formula (2): 
His-Ser-Asp-Ala-Val-Phe-Thr-R1-R2-Tyr-Thr-R3-Leu-Arg-Lys-Gln-
Leu-Ala-R4-R5-Lys-Tyr-Leu-R6-R7-R8-R9-R10-Hse (SEQ ID NO: 2)
wherein the symbols R₁ to R₁₀ are as defined above and Hse is a homoserine or homoserine lactone residue, can be prepared by treating the corresponding fused proteins mentioned above with cyanogen bromide.

The recombinant plasmids can be prepared by per se known techniques except that the DNA to be inserted encodes a polypeptide comprising the above-mentioned leader peptide having linked thereto in a downstream direction one molecule or a plurality of molecules arranged in tandem of the above-mentioned peptide of general formula (1) (SEQ ID NO: 1). The transformant microorganisms can be obtained by introducing one of the recombinant plasmids into a microorganism by per se known techniques. As examples of the microorganism that can be used, there may be mentioned the Escherichia coli strains JM109 and HB101 and the like.

### BRIEF DESCRIPTION OF THE DRAWINGS

Fig. 1 is a flowchart illustrating a scheme for inserting a synthetic gene coding for [Leu¹⁷]-VIP-Met (SEQ ID NO: 3) into a plasmid.

Fig. 2 is a flowchart illustrating a scheme for attaining a repetition of the synthetic gene coding for [Leu¹⁷]-VIP-Met (SEQ ID NO: 3) in the plasmid.

Fig. 3 is a flowchart illustrating a scheme for inserting the synthetic gene coding for [Leu¹⁷]-VIP-Met (SEQ ID NO: 3) downstream from the Sarcophaga lectin gene.

Fig. 4 is a flowchart illustrating a scheme for connecting the gene coding for [Leu¹⁷]-VIP-Met (SEQ ID No: 3) and Sarcophaga lectin to the tac promoter downstream from the promoter.

Fig. 5 is a flowchart illustrating a scheme for parting the gene for Sarcophaga lectin, the leader peptide source, by utilizing the replication chain reaction.

Fig. 6 is a graphic representation of the results obtained in an assay of the inhibitory activity of [Leu¹⁷]-VIP-Hse against histamine-induced bronchoconstriction.

### DETAILED DESCRIPTION OF THE INVENTION

The following VIP analog production examples and physiological activity test examples illustrate the invention in further detail.

The following production examples deal exclusively with the preparation of [Leu¹⁷]-VIP-Hse (inclusive of homoserine lactone) and VIP#10-Hse (SEQ ID NO: 30). The VIP analogs covered by the present invention can be prepared in the same manner since the genes therefor are synthetic ones.

### PRODUCTION EXAMPLE 1

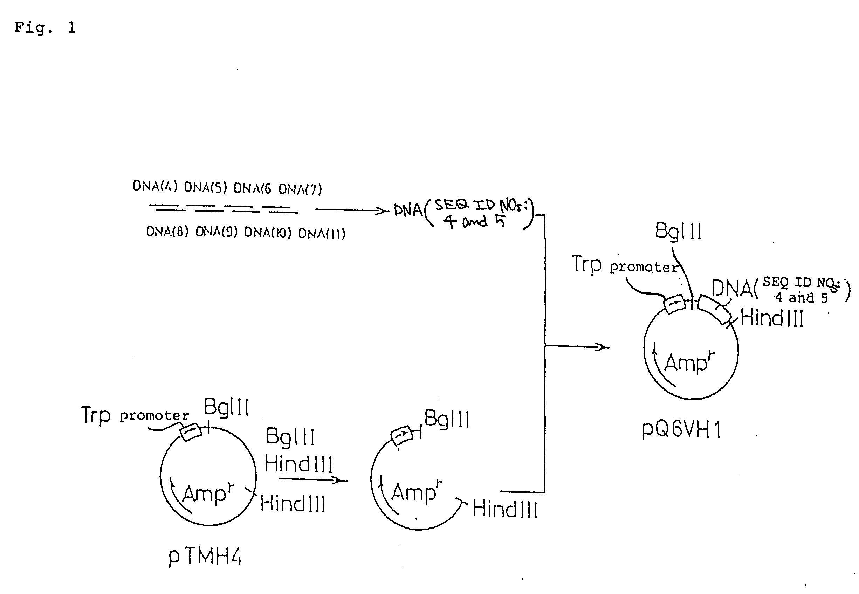

### a) Step 1: Synthesis of [Leu¹⁷]-VIP-Met gene (cf. Fig. 1)

His-Ser-Asp-Ala-Val-Phe-Thr-Asp-Asn-Tyr-Thr-Arg-Leu-Arg-Lys-
Gln-Leu-Ala-Val-Lys-Lys-Tyr-Leu-Asn-Ser-Ile-Leu-Asn-Met

For preparing the polypeptide shown above (SEQ ID NO:3), a double-stranded DNA fragment (SEQ ID NOs:4 and 5) having the nucleotide sequence shown below in terms of formula (3) was produced. 
Formula (3) (SEQ ID NOs: 4 and 5)

1        10        20        30        40        50        60
5'-GATCTTCATGCATTCAGATGCAGTGTTCACTGACAACTACACCCGCCTGCGCAAACAGCT
3'-    AAGTACGTAAGTCTACGTCACAAGTGACTGTTGATGTGGGCGGACGCGTTTGTCGA

         70        80        90        100       110
GGCTGTAAAGAAATACCTGAACTCAATATTGAACATGGGGATCCTGTGATA    -3'
CCGACATTTCTTTATGGACTTGAGTTATAACTTGTACCCCTAGGACACTATTCGA-5'
The double-stranded DNA shown by the above formula (3) (SEQ ID NOs: 4 and 5) was prepared in the following manner.

First, single-stranded DNAs respectively having the nucleotide sequences shown below by formulas (4) to (11) (SEQ ID NOs: 6 to 13) were synthesized using a DNA synthesizer ("Gene Assembler", Pharmacia). Among these single-stranded DNAs, (4) (SEQ ID NO: 6) and (8) (SEQ ID NO: 10), (5) (SEQ ID NO: 7) and (9) (SEQ ID NO: 11), (6) (SEQ ID NO: 8) and (10) (SEQ ID NO: 12), and (7) (SEQ ID NO: 9) and (11) (SEQ ID NO: 13) are complementary to each other except for minor terminal portions and can form double-stranded DNAs, respectively.
Formula (4) (SEQ ID NO: 6)
5'-GATCTTCATGCATTCAGATGCAGTGTT-3'
Formula (5) (SEQ ID NO: 7)
5'-CACTGACAACTACACCCGCCTGCGCAAA-3'
Formula (6) (SEQ ID NO: 8)
5'-CAGCTGGCTGTAAAGAAATACCTGAACT-3'
Formula (7) (SEQ ID NO: 9)
5'-CAATATTGAACATGGGGATCCTGTGATA-3'
Formula (8) (SEQ ID NO: 10)
5'-TCAGTGAACACTGCATCTGAATGCATGAA-3'
Formula (9) (SEQ ID NO: 11)
5'-CCAGCTGTTTGCGCAGGCGGGTGTAGTTG-3'
Formula (10) (SEQ ID NO: 12)
5'-ATATTGAGTTCAGGTATTTCTTTACAG-3'
Formula (11) (SEQ ID NO: 13)
5'-AGCTTATCACAGGATCCCCATGTTCA-3'
The above single-stranded DNAs of formulas (4) to (11) (SEQ ID NOs: 6 to 13) were respectively phosphorylated at the 5' terminus by treatment with polynucleotide kinase. Then, the complementary DNA pairs, (4) (SEQ ID NO: 6) and (8) (SEQ ID NO: 10), (5) (SEQ ID NO: 7) and (9) (SEQ ID NO: 11), (6) (SEQ ID NO: 8) and (10) (SEQ ID NO: 12), and (7) (SEQ ID NO: 9) and (11) (SEQ ID NO: 13), were respectively subjected to annealing and the resulting four double-stranded DNAs were connected together in tandem by treating with DNA ligase to give the double-stranded DNA fragment of formula (3) (SEQ ID NOs: 4 and 5). This synthetic DNA codes for Ile-Phe-Met and [Leu¹⁷]-VIP-Met-Gly-Ile-Leu which follows (SEQ ID NO: 14).

### b) Step 2: Insertion of the DNA coding for [Leu¹⁷]-VIP-Met (SEQ ID NO: 3) into a vector and attaining repetition of the [Leu¹⁷]-VIP-Met molecular portion

Referring to Fig. 1, a procedure for the insertion of the synthetic DNA obtained in Step 1 into an expression vector is described below.

The plasmid described in JP-A-3-80096 (pTMH4; this plasmid contains four molecules, arranged in tandem, of a DNA coding for [Leu¹³]-motilin-Met and a transformant strain derived from Escherichia coli HB101 by transformation with the recombinant plasmid has been deposited with National Institute of Bioscience and Human Technology (formerly, Fermentation Research Institute), Agency of Industrial Science and Technology under the Budapest Treaty (deposit number FERM BP-2555) was cleaved with the restriction enzymes Bg1II and HindIII and the larger fragment was purified. This was ligated with the DNA fragment of formula (3) (SEQ ID NOs: 4 and 5) to reconstruct a plasmid. This plasmid was named pQ6VH1.

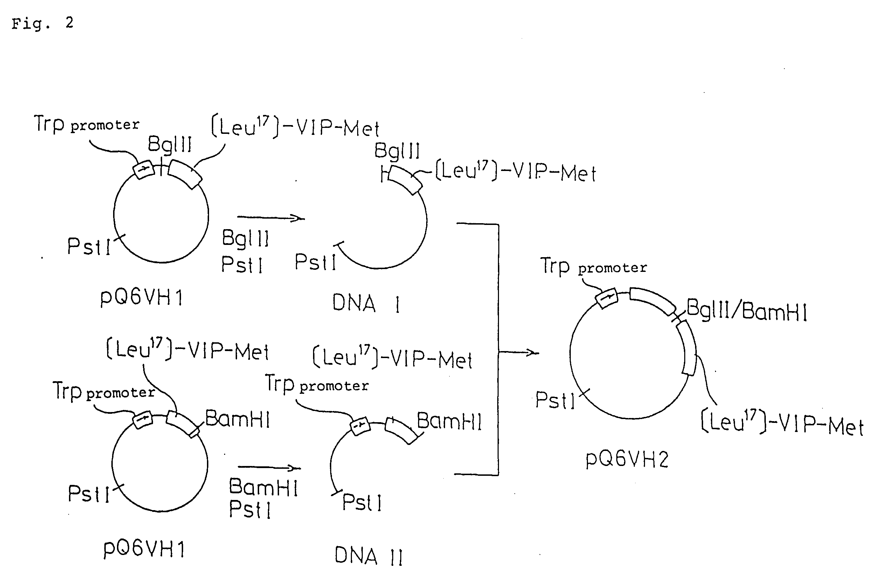

Now, referring to Fig. 2, a procedure for connecting a plurality of [Leu¹⁷]-VIP-Met molecules in tandem is explained below. The plasmid pQ6VH1 was cleaved with the restriction enzymes PstI and Bg1II and a DNA fragment containing the [Leu¹⁷]-VIP-Met gene was purified (DNA I). Separately, pQ6VH1 was cleaved with the restriction enzymes PstI and BamHI and a DNA fragment containing the [Leu¹⁷]-VIP-Met gene was purified (DNA II). DNA I and DNA II were ligated together to reconstruct a plasmid, pQ6VH2, containing two [Leu¹⁷]-VIP-Met gene molecules connected in tandem. On that occasion, the Bg1II and BamHI cleavage sites are connected with each other but the ligation site is no more a recognition site for both the enzymes. Therefore, by repeating the above cleavage and ligation procedures, a plasmid containing an arbitrarily chosen number of [Leu¹⁷]-VIP-Met gene molecules linked in tandem can be obtained.

In this way, plasmids containing 3 to 10 [Leu¹⁷]-VIP-Met gene molecules linked in tandem, respectively named pQ6VH3, pQ6VH4, pQ6VH5, pQ6VH6, pQ6VH7, pQ6VH8, pQ6VH9 and pQ6VH10, were obtained.

### c) Step 3: Insertion of the gene for Sarcophaga lectin, the leader peptide source, into a vector

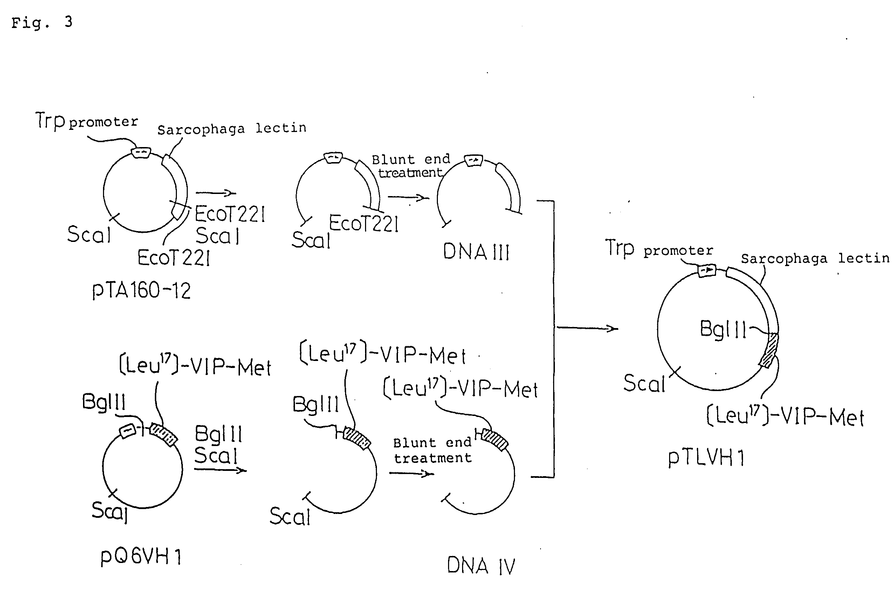

Referring to Fig. 3, a procedure for further introduction of the gene for Sarcophaga lectin, the leader peptide source, into the recombinant plasmid obtained in Step 2 is now explained below.

A plasmid (pTA160) useful for the expression of Sarcophaga lectin [Journal of Biological Chemistry, vol. 260, pages 12228-12233 (1985)] can be made, for example, the following processes:
The plasmid pKK223-3 (Pharmacia) was cleaved with restriction enzymes TagI and VspI to obtain a DNA fragment having the Tac promoter. The following DNA-1 (SEQ ID NOs: 15 and 16) and DNA-2 (SEQ ID NOs: 17 and 18) for a tryptophan operon derived from Escherichia coli were synthesized by a DNA synthesizer and then linked to the above DNA fragment to obtain the following DNA-3 (SEQ ID NOs: 19 and 20). 
DNA-1 (SEQ ID NOs: 15 and 16)

1          10         20         30         40         50         60
5'-AATTAATTCCATTCAACGCCAGTCCCGAACGTGAAATTTCCTCTCTTGCTGGCGCGATTG
3'-     TTAAGGTAAGTTGCGGTCAGGGCTTGCACTTTAAAGGAGAGAACGACCGCGCTAAC

70         80         90         100
CAGCTGTGGTGTCATGGTCGGTGATCGCCAGGGTGCCGACGCGCATCT   -3'
GTCGACACCACAGTACCAGCCACTAGCGGTCCCACGGCTGCGCGTAGAGC-5'

DNA-2 (SEQ ID NOs: 17 and 18)

1          10         20         30         40         50
5'-TAATCATCGAACTAGTTAACTAGTACGCAATAACTCAATCTAGAACGGAGGTTTG      -3'
3'-   TAGTAGCTTGATCAATTGATCATGCGTTATTGAGTTAGATCTTGCCTCCAAACTTAA-5'

DNA-3 (SEQ ID NOs: 19 and 20)

1          10         20         30         40         50         60
5'-AATTAATTCCATTCAACGCCAGTCCCGAACGTGAAATTTCCTCTCTTGCTGGCGCGATTG
3'-     TTAAGGTAAGTTGCGGTCAGGGCTTGCACTTTAAAGGAGAGAACGACCGCGCTAAC

70         80         90         100        110        120
CAGCTGTGGTGTCATGGTCGGTGATCGCCAGGGTGCCGACGCGCATCTCGACTGCACGGT
GTCGACACCACAGTACCAGCCACTAGCGGTCCCACGGCTGCGCGTAGAGCTGACGTGCCA
 
130 140 150 160 170 180
GCACCAATGCTTCTGGCGTCAGGCAGCCATCGGAAGCTGTGGTATGGCTGTGCAGGTCGT
CGTGGTTACGAAGACCGCAGTCCGTCGGTAGCCTTCGACACCATACCGACACGTCCAGCA

190 200 210 220 230 240
AAATCACTGCATAATTCGTGTCGCTCAAGGCGCACTCCCGTTCTGGATAATGTTTTTTGC
TTTAGTGACGTATTAAGCACAGCGAGTTCCGCGTGAGGGCAAGACCTATTACAAAAAACG

250 260 270 280 290 300
GCCGACATCATAACGGTTCTGGCAAATATTCTGAAATGAGCTGTTGACAATTAATCATCG
CGGCTGTAGTATTGCCAAGACCGTTTATAAGACTTTACTCGACAACTGTTAATTAGTAGC

310 320 330 340
AACTAGTTAACTAGTACGCAATAACTCAATCTAGAACGGAGGTTTG -3'
TTGATCAATTGATCATGCGTTATTGAGTTAGATCTTGCCTCCAAACTTAA-5'
The plasmid pBR322 (Toyobo Co., Ltd.) was cleaved with restriction enzyme EcoRI and then linked to the above DNA-3 fragment to obtain plasmid pTM1. A DNA fragment, which was prepared by PCR (polymerase chain reaction) using cDNA clone described in J.Biol. Chem., Vol. 260, pp. 12228-12233 (1985) and primers (a) and (b) (SEQ ID NOs: 21 and 22), was treated with restriction enzymes EcoRI and HindIII to obtain DNA-4 (SEQ ID NOs: 23 and 24).
Primer (a) (SEQ ID NO: 21)
5'-TTGAATTCATGGTGCCCCAATTACAAAAGGCT-3'
Primer (b) (SEQ ID NO: 22)
5'-GAAGCTTTTAACAAATTTCATTATTTTCTGC-3' 
DNA-4 (SEQ ID NOs: 23 and 24)

1 10 20 30 40 50 60
5'-AATTCATGGTGCCCCAATTACAAAAGGCTTTAGATGGCAGAGAATATCTTATTGAAACAG
3'- GTACCACGGGGTTAATGTTTTCCGAAATCTACCGTCTCTTATAGAATAACTTTGTC

70 80 90 100 110 120
AACTTAAGTACAATTGGCATCAAGCCTGGCATGAATGTGCCCGCCATGATCAACAACTTG
TTGAATTCATGTTAACCGTAGTTCGGACCGTACTTACACGGGCGGTACTAGTTGTTGAAC

130 140 150 160 170 180
TAACAATCGAAAGTGCTGATAAGAATAATGCCATTATCGATCTGGTGAAACGGGTCGTTG
ATTGTTAGCTTTCACGACTATTCTTATTACGGTAATAGCTAGACCACTTTGCCCAGCAAC

190 200 210 220 230 240
GAAAATCTCATAATTTATGGTTGGGCGGCAATGATGAGTATAGTTCAAGTCGTGACTATG
CTTTTAGAGTATTAAATACCAACCCGCCGTTACTACTCATATCAAGTTCAGCACTGATAC

250 260 270 280 290 300
GCAGACCCTTTTTCTGGTCACCCACCGGTCAAGCATTCTCCTTTGCCTACTGGTCGGAAA
CGTCTGGGAAAAAGACCAGTGGGTGGCCAGTTCGTAAGAGGAAACGGATGACCAGCCTTT

310 320 330 340 350 360
ACAATCCCGATAATTATAAGCATCAAGAACATTGTGTCCATATATGGATACAAAGCCCT
TGTTAGGGCTATTAATATTCGTAGTTCTTGTAACACAGGTATATACCTATGTTTCGGGA

370 380 390 400 410 420
TATATCAATGGAACGATAATGATTGTAATGTTAAAATGGGTTACATATGTGAACCCAATC
ATATAGTTACCTTGCTATTACTAACATTACAATTTTACCCAATGTATACACTTGGGTTAG

430 440 450 460 470 480
ATTTCCGGGAAACATATGATCAAGCACTCAAGCAAAAATGCGAAGCAATTAAGATAACAA
TAAAGGCCCTTTGTATACTAGTTCGTGAGTTCGTTTTTACGCTTCGTTAATTCTATTGTT

490 500 510 520 530 540
ATTCAAAAATTTCAACAGAATTTGATCAATTGCATGCCAAACAATCATTGGAATTTGATA
TAAGTTTTTAAAGTTGTCTTAAACTAGTTAACGTACGGTTTGTTAGTAACCTTAAACTAT

550 560 570 580 590 600
GTATAACGCAAAATGTAGCAAAAGTGAATGAAGATTGGAAAATTGAAATCCAAAAACTAC
CATATTGCGTTTTACATCGTTTTCACTTACTTCTAACCTTTTAACTTTAGGTTTTTGATG
 
610 620 630 640 650 660
AGAATGCCACACAAATTGCCATACAACAGATTATGGAAAATCATGAGAAGAAGATAAGAG
TCTTACGGTGTGTTTAACGGTATGTTGTCTAATACCTTTTAGTACTCTTCTTCTATTCTC

670 680 690 700 710 720
ATTTAAGTGATAATCTACTTAAGCAGCTACAAGATTCCAATGAACAACTGAAACAGTCCA
TAAATTCACTATTAGATGAATTCGTCGATGTTCTAAGGTTACTTGTTGACTTTGTCAGGT

730 740 750 760 770 780
CTGACCATATGAATGCATCGTTTGGTGAGAAATTGAAAGGCCAACAAGCAGAAAATAATG
GACTGGTATACTTACGTAGCAAACCACTCTTTAACTTTCCGGTTGTTCGTCTTTTATTAC

790
AAATTTGTTAAA -3′
TTTAAACAATTTTCGA-5′
The above plasmid pTM1 was treated with restriction enzymes EcoRI and HindIII, and the resulting large fragment was linked to the above DNA-4 (SEQ ID NOs: 23 and 24) to obtain the plasmid pTA160.

This plasmid (pTA160) was cleaved with the restriction enzymes EcoT221 and ScaI and a DNA fragment was purified. This DNA fragment was rendered blunt-ended by treatment with a Takara Shuzo blunting kit (the resultant DNA fragment is referred to as DNA III). Separately, the plasmid pQ6VH1 was cleaved with the restriction enzymes ScaI and Bg1II, followed by blunting treatment in the same manner as mentioned above to give DNA IV. DNA III and DNA IV were ligated together using DNA ligase, whereby a plasmid, pTLVH1, containing a DNA coding for a polypeptide resulting from linking one molecule of [Leu¹⁷]-VIP-Met to a polypeptide composed of the 24th to 265th amino acid residues of Sarcophaga lectin was constructed.

Furthermore, pQ6VH2, pQ6VH3, pQ6VH4, pQ6VH5, pQ6VH6, pQ6VH7, pQ6VH8, pQ6VH9 and pQ6VH10 were treated in the same manner, namely subjected to cleavage with the restriction enzymes ScaI and Bg1II, blunting and ligation of the respective DNA fragments to DNA III, to give plasmids, pTLVH2, pTLVH3, pTLVH4, pTLVH5, pTLVH6, pTLVH7, pTLVH8, pTLVH9 and pTLVH10, containing a DNA coding for a polypeptide composed of a polypeptide consisting of the 24th to 265th amino acid residues of Sarcophaga lectin and 2 to 10 molecules of [Leu¹⁷]-VIP-Met arranged in tandem and connected to the lectin-derived polypeptide.

### d) Step 4: Construction of a plasmid for fused protein expression

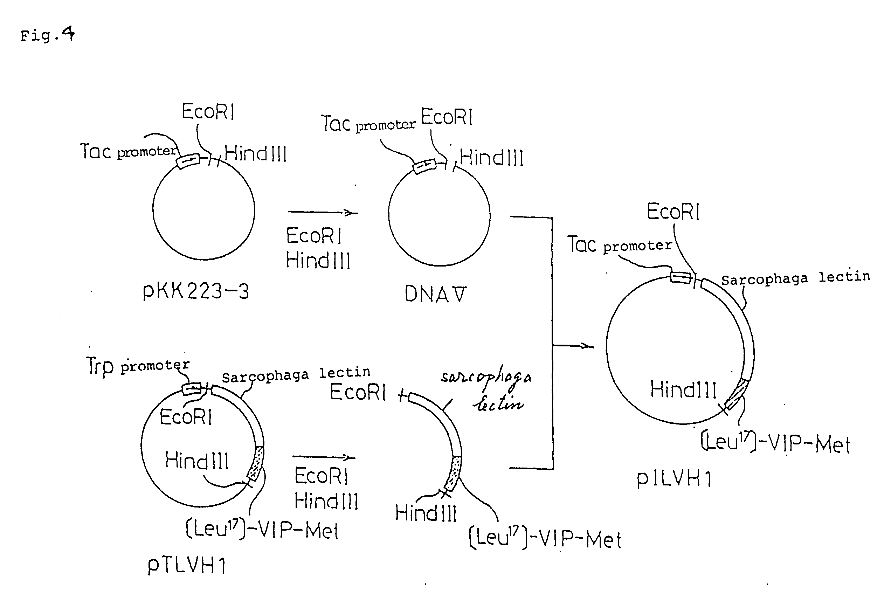

Referring to Fig. 4, the construction of a plasmid for fused protein expression is described below. The plasmid pKK223-3 (Pharmacia) was cleaved with the restriction enzymes EcoRI and HindIII to give DNA V. The plasmid pKK223-3 is a plasmid vector having the tac promoter and capable of expressing a protein encoded by an exogenous gene in the form of an isopropyl-1-thio-β-D-galactoside (IPTG). Separately, pTLVH1 was cleaved with the restriction enzymes EcoRI and HindIII and the VIP analog-encoding fragment was ligated to DNA V to give an expression vector, pILVH1, containing the tac promoter and a gene subsequent thereto for coding for a polypeptide resulting from linking one molecule of [Leu¹⁷]-VIP-Met to a polypeptide composed of the 24th to 265th amino acid residues of Sarcophaga lectin.

The plasmids pTLVH2, pTLVH3, pTLVH4, pTLVH5, pTLVH6, pTLVH7, pTLVH8, pTLVH9 and pTLVH10 were subjected to the same treatment steps, namely cleavage with the restriction enzymes EcoRI and HindIII, isolation of the VIP analog-encoding DNA and ligation to DNA V to give expression plasmids, pILVH2, pILVH3, pILVH4, pILVH5, pILVH6, pILVH7, pILVH8, pILVH9 and pILVH10, containing the tac promoter and a gene subsequent thereto for coding for a polypeptide resulting from linking two to ten molecules of [Leu¹⁷]-VIP-Met arranged in tandem to a polypeptide composed of the 24th to 265th amino acid residues of Sarcophaga lectin.

### e) Step 5: Paring of the gene for Sarcophaga lectin for providing a gene for a leader peptide

For producing [Leu¹⁷]-VIP-Hse in large quantities, it is desirable to increase the proportion of [Leu¹⁷]-VIP-Met in the protein expressed as a fused protein and to select a plasmid capable of high level expression of the fused protein. Therefore, the Sarcophaga lectin gene portion was pared by the replication chain reaction method for providing a gene for an appropriate leader peptide.

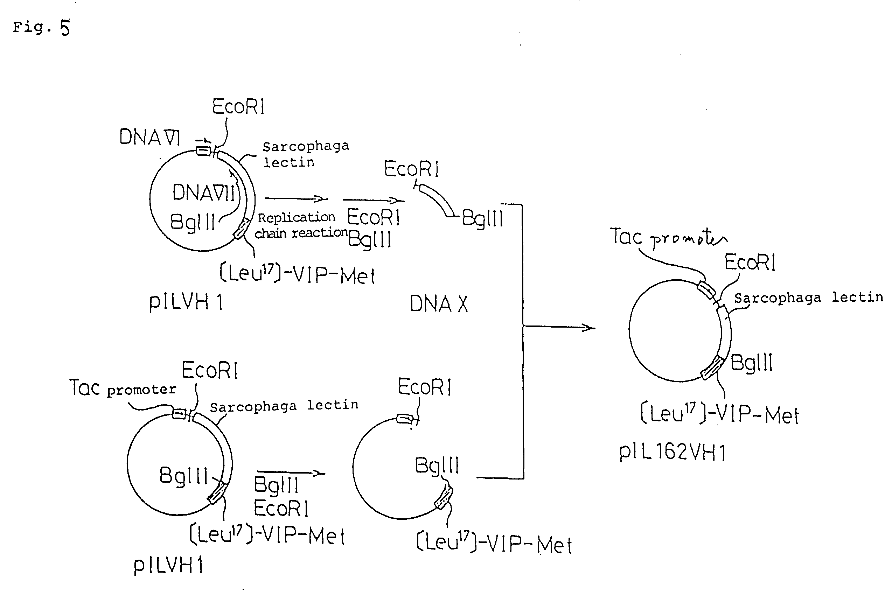

This process is now explained referring to Fig. 5. As primers for carrying out the replication chain reaction, the following DNAs were synthesized: a DNA (DNA VI, SEQ ID NO: 25) corresponding to the sequence covering the 39th to 55th nucleotides of pKK223-3, an antisense DNA (DNA VII, SEQ ID NO: 26) to a DNA resulting from addition of a nucleotide sequence recognizable by the restriction enzyme Bg1II to a DNA corresponding to the sequence covering the 162nd to 178th nucloetides of the Sarcophaga lectin-encoding DNA, an antisense DNA (DNA VIII, SEQ ID NO: 27) to a DNA resulting from addition of a nucleotide sequence recognizable by the restriction enzyme Bg1II to a DNA corresponding to the sequence covering the 288th to 304th nucloetides of the Sarcophaga lectin-encoding DNA, an antisense DNA (DNA IX, SEQ ID NO: 28) to a DNA resulting from addition of a nucleotide sequence recognizable by the restriction enzyme Bg1II to a DNA corresponding to the sequence covering the 496th to 513th nucloetides of the Sarcophaga lectin-encoding DNA, and an antisense DNA (DNA XIII, SEQ ID NO: 29) to a DNA resulting from addition of a nucleotide sequence recognizable by the restriction enzyme Bg1II to a DNA corresponding to the sequence covering the 135th to 151st nucleotides of the Sarcophaga lectin-encoding DNA.
DNA VI (SEQ ID NO: 25)
5'-GCTCGTATAATGTGTGG-3'
DNA VII (SEQ ID NO: 26)
5'-CCAGATCTGTTGTTGATCATGGCGG-3'
DNA VIII (SEQ ID NO: 27)
5'-CCAGATCTTGCCATAGTCACGACTT-3'
DNA IX (SEQ ID NO: 28)
5'-CCAGATCTTGAGTGCTTGATCATA-3'
DNA XIII (SEQ ID NO: 29)
5'-CCAGATCTGCCAGGCTTGATGCCAA-3'
Following addition of pILVH1 as a template DNA and DNA VI (SEQ ID NO: 25) and DNA VII (SEQ ID NO: 26) as primer DNAs, the replication chain reaction was carried out. After the reaction, the product DNA was isolated from the reaction mixture and cleaved with the restriction enzymes EcoRI and Bg1II, and DNA X was isolated. This DNA X codes for a polypeptide corresponding to the 24th to 59th amino acid residues of Sarcophaga lectin. Separately, pILVH1 was cleaved with the restriction enzymes EcoRI and Bg1II, the VIP analog-encoding DNA fragment was isolated and ligated to DNA X to give an expression plasmid, pIL162VH1, coding for a polypeptide resulting from connection of one molecule of [Leu¹⁷]-VIP-Met to a polypeptide corresponding to the 24th to 59th amino acid residues of Sarcophaga lectin.

The plasmids pILVH2, pILVH3, pILVH4, pILVH5, pILVH6, pILVH7, pILVH8, pILVH9 and pILVH10 were subjected to the same treatment steps, namely cleavage with the restriction enzymes EcoRI and Bg1II, isolation of the VIP analog-encoding DNA and ligation to DNA X, to give plasmids, pIL162VH2, pIL162VH3, pIL162VH4, pIL162VH5, pIL162VH6, pIL162VH7, pIL162VH8, pIL162VH9 and pIL162VH10, containing the tac promoter and a gene subsequent thereto for coding for a polypeptide resulting from linking two to ten [Leu¹⁷]-VIP-Met molecules arranged in tandem to a polypeptide corresponding to the 24th to 59th amino acid residues of Sarcophaga lectin.

Then, following addition of pILVH1 as a template DNA and DNA VI (SEQ ID NO: 25) and DNA VIII (SEQ ID NO: 27) as primer DNAs, the replication chain reaction was carried out. The product DNA was isolated from the reaction mixture and cleaved with the restriction enzymes EcoRI and Bg1II, and DNA XI was isolated. This DNA IX codes for a polypeptide corresponding to the 24th to 101st amino acid residues of Sarcophaga lectin.

Separately, pILVH1 was cleaved with the restriction enzymes EcoRI and Bg1II, and the VIP analog-encoding DNA was isolated and ligated to DNA XI to give an expression plasmid, pIL288VH1, coding for a polypeptide resulting from linking one [Leu¹⁷]-VIP-Met molecule to a polypeptide corresponding to the 24th to 101st amino acid residues of Sarcophaga lectin.

The plasmids pILVH2, pILVH3, pILVH4, pILVH5, pILVH6, pILVH7, pILVH8, pILVH9 and pILVH10 were also subjected to the same treatment steps, namely cleavage with the restriction enzymes EcoRI and Bg1II, isolation of the VIP analog-encoding DNA and ligation to DNA XI, to give plasmids, pIL288VH2, pIL288VH3, pIL288VH4, pIL288VH5, pIL288VH6, pIL288VH7, pIL288VH8, pIL288VH9 and pIL288VH10, containing the tac promoter and a gene subsequent thereto for coding for a polypeptide resulting from linking two to ten [Leu¹⁷]-VIP-Met molecules arranged in tandem to a polypeptide corresponding to the 24th to 101st amino acid residues of Sarcophaga lectin.

Further, the replication chain reaction was carried out following addition of pILVH1 as a template DNA and DNA VI (SEQ ID NO: 25) and DNA IX (SEQ ID NO: 28) as primer DNAs. The product DNA was isolated from the reaction mixture and cleaved with the restriction enzymes EcoRI and Bg1II, and DNA XII was isolated. This DNA XII codes for a polypeptide corresponding to the 24th to 171st amino acid residues of Sarcophaga lectin.

Separately, pILVR1 was cleaved with the restriction enzymes EcoRI and Bg1II, and the VIP analog-encoding DNA was isolated and ligated to DNA XII to give an expression plasmid, pIL496VH1, coding for a polypeptide resulting from linking one [Leu¹⁷]-VIP-Met molecule to a polypeptide corresponding to the 24th to 171st amino acid residues of Sarcophaga lectin.

The plasmids pILVH2, pILVH3, pILVH4, pILVH5, pILVH6, pILVH7, pIlVH8, pILVH9 and pILVH10 were also subjected to the same treatment steps, namely cleavage with the restriction enzymes EcoRI and Bg1II, and isolation of the VIP analog-encoding DNA and ligation to DNA XII, to give plasmids, pIL496VH2, pIL496VH3, pIL496VH4, pIL496VH5, pIL496VH6, pIL496VH7, pIL496VH8, pIL496VH9 and pIL496VH10, containing the tac promoter and a gene subsequent thereto for coding for a polypeptide resulting from linking two to ten [Leu¹⁷]-VIP-Met molecules arranged in tandem to a polypeptide corresponding to the 24th to 171st amino acid residues of Sarcophaga lectin.

Further, the replication chain reaction was carried out following addition of pILVH1 as a template DNA and DNA VI (SEQ ID NO: 25) and DNA XIII (SEQ ID NO: 29) as primer DNAs. The product DNA was isolated from the reaction mixture and cleaved with the restriction enzymes EcoRI and Bg1II, and DNA XIV was isolated. This DNA XIV codes for a polypeptide corresponding to the 24th to 50th amino acid residues of Sarcophaga lectin.

Separately, pILVH1 was cleaved with the restriction enzymes EcoRI and Bg1II, and the VIP analog-encoding DNA was isolated and ligated to DNA XIV to give an expression plasmid, pIL135VH1, coding for a polypeptide resulting from linking one [Leu¹⁷]-VIP-Met molecule to a polypeptide corresponding to the 24th to 50th amino acid residues of Sarcophaga lectin.

The plasmids pILVH2, pILVH3, pILVH4, pILVH5, pILVH6, pILVH7, pILVH8, pILVH9 and pILVH10 were also subjected to the same treatment steps, namely cleavage with the restriction enzymes EcoRI and Bg1II, isolation of the VIP analog-encoding DNA and ligation to DNA XIV, to give plasmids, pIL135VH2, pIL135VH3, pIL135VH4, pIL135VH5, pIL135VH6, pIL135VH7, pIL135VH8, pIL135VH9 and pIL135VH10, containing the tac promoter and a gene subsequent thereto for coding for a polypeptide resulting from linking two to ten [Leu¹⁷]-VIP-Met molecules arranged in tandem to a polypeptide corresponding to the 24th to 50th amino acid residues of Sarcophaga lectin.

### f) Step 6: Fused protein production

Transformants were produced by allowing the Escherichia coli strain JM109 to take up the plasmids pQ6VH1, pQ6VH2, pQ6VH3, pQ6VH4, pQ6VH5, pQ6VH6, pQ6VH7, pQ6VH8, pQ6VH9 and pQ6VH10, respectively. These transformants were cultured in a medium containing 50 µg/ml of ampicillin at 25° to 40°C under aeration. When the A₅₅₀ value reached 0.4 to 5.0, IPTG was added to a final concentration of 1 mM. Then, cultivation was continued for 5 to 24 hours, followed by recovery of cells by centrifugation (5,000 rpm, 4°C, 20 minutes).

The cells were sampled and analyzed by 12.5% polyacrylamide gel electrophoresis. The bands of the desired proteins were detected only in trace quantities in the expected positions. These bands were measured by a densitometer, whereupon the expression of the respective proteins were found to be not more than 1% of the whole protein in each case.

The following plasmids for fused protein expression as obtained in Step 5 were used to transform Escherichia coli JM109: pILVH1, pILVH2, pILVH3, pILVH4, pILVH5, pILVH6, pILVH7, pILVH8, pILVH9, pILVH10, pIL135VH1, pIL135VH2, pIL135VH3, pIL135VH4, pIL135VH5, pIL135VH6, pIL135VH7, pIL135VH8, pIL135VH9, pIL135VH10, pIL162VH1, pIL162VH2, pIL162VH3, pIL162VH4, pIL162VH5, pIL162VH6, pIL162VH7, pIL162VH8, pIL162VH9, pIL162VH10, pIL288VH1, pIL288VH2, pIL288VH3, pIL288VH4, pIL288VH5, pIL288VH6, pIL288VH7, pIL288VH8, pIL288VH9, pIL288VH10, pIL496VH1, pIL496VH2, pIL496VH3, pIL496VH4, pIL496VH5, pIL496VH6, pIL496VH7, pIL496VH8, pIL496VH9 and pIL496VH10. Among the resulting Escherichia coli transformants, the one harboring pIL496VH4 has been deposited with National Institute for Bioscience and Human Technology (formerly, Fermentation Research Institute), Agency of Industrial Science and Technology under the Budapest Treaty (deposit number: FERM BP-4583).

Using a type D fermenter (Able Corp.), these transformants were each cultured in a medium (e.g. L medium, amino acid-containing M9 medium) containing 50 µg/ml of ampicillin at 25° to 40°C (preferably between 30° to 38°C) under aeration. When the A₅₅₀ value reached 0.4 to 5.0 (preferably 0.6 to 0.8), IPTG was added to a final concentration of 1 mM. Cultivation was then continued for 5 to 24 hours. Cells were harvested by centrifugation (5,000 rpm, 4°C, 20 minutes). The cells were sampled and analyzed by 12.5% SDS polyacrylamide gel electrophoresis. A band was detected at the position corresponding to the molecular weight of each desired fused protein. Densitometry of the bands revealed the levels of expression of the desired fused proteins as shown below in Table 1.

Table 1 has no data for recombinant plasmids pIL135VH1 to pIL135VH10 since the transformants containing these plasmids failed to express the desired fused proteins.

**TABLE 1**

| Plasmid | Proportion of fused protein to total cell protein (%) | Plasmid | Proportion of fused protein to total cell protein (%) |
|---|---|---|---|
| pILVH1 | <2 | pIL288VH1 | <2 |
| pILVH2 | <2 | pIL288VH2 | <2 |
| pILVH3 | <2 | pIL288VH3 | <2 |
| pILVH4 | 15 | pIL288VH4 | 13 |
| pILVH5 | <2 | pIL288VH5 | <2 |
| pILVH6 | <2 | pIL288VH6 | <2 |
| pILVH7 | <2 | pIL288VH7 | <2 |
| pILVH8 | <2 | pIL288VH8 | <2 |
| pILVH9 | <2 | pIL288VH9 | <2 |
| pILVH10 | <2 | pIL288VH10 | <2 |
| pIL162VH1 | <2 | pIL496VH1 | <2 |
| pIL162VH2 | <2 | pIL496VH2 | <2 |
| pIL162VH3 | <2 | pIL496VH3 | <2 |
| pIL162VH4 | 10 | pIL496VH4 | 25 |
| pIL162VH5 | <2 | pIL496VH5 | <2 |
| pIL162VH6 | <2 | pIL496VH6 | <2 |
| pIL162VH7 | <2 | pIL496VH7 | <2 |
| pIL162VH8 | <2 | pIL496VH8 | <2 |
| pIL162VH9 | <2 | pIL496VH9 | <2 |
| pIL162VH10 | <2 | pIL496VH10 | <2 |

### g) Step 7: Isolation and purification of [Leu¹⁷]-VIP-Hse

The Escherichia coli strain JM109 was transformed with the recombinant plasmid pIL496VH4 obtained in the above-mentioned step 5 and cultured for fused protein production in the presence of IPTG (cf. Step 6). To the cell precipitate (corresponding to 300 ml of medium) was added 8 ml of purified water for suspending the precipitate and the suspension was subjected to sonication to disrupt the cells. The precipitate fraction containing a [Leu¹⁷]-VIP-Met-containing inclusion body was recovered by centrifugation (5,000 rpm, 4°C, 15 minutes), 40 ml of 70% formic acid was added to the precipitate for dissolving the same, 0.1 g of cyanogen bromide was then added, and the reaction was carried out at 30°C for 16 hours. After completion of the reaction, 160 ml of purified water was added, the mixture was centrifuged (5,000 rpm, 4°C, 15 minutes), and the supernatant was lyophilized to give a lyophilizate powder which contained [Leu¹⁷]-VIP-Hse.

To this was added 10 ml of 10% acetic acid and the resulting solution was subjected to liquid chromatography using a Waters µ-Bondapak C-18 column (19 mm × 15 cm) for the purification of [Leu¹⁷]-VIP-Hse.

### Purification conditions:

Eluent: 0.012 N hydrochloric acid (linear acetonitrile gradient from 15% to 50%, 30 minutes);
Flow rate: 7.0 ml/minute
A peak fraction corresponding to [Leu¹⁷]-VIP-Hse was recovered and lyophilized, the lyophilizate was sampled and analyzed using an Applied Biosystems peptide sequencer. It was thus confirmed that [Leu¹⁷]-VIP-Hse had been correctly prepared.

The above procedure gave 30 to 40 mg of [leu¹⁷]-VIP-Hse per liter of Escherichia coli culture medium (this yield is about 10 to 100 times higher as compared with the yield of about several hundred micrograms, at most about several milligrams, per liter generally obtainable by the prior art technology).

### PHYSIOLOGICAL ACTIVITY TEST EXAMPLE 1

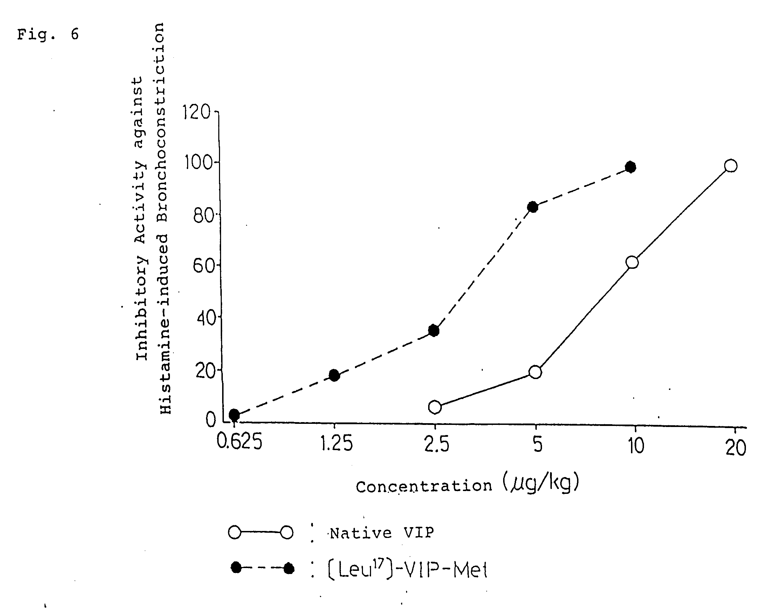

The [Leu¹⁷]-VIP-Hse obtained in the above manner was used as the test sample. Using native VIP as a control, the sample was tested by the Konzett-Roessler method using guinea pigs [Journal of Biological Chemistry, vol. 256, pages 6389-6392 (1991)] for confirming that the test sample had inhibitory activity against histamine-induced bronchoconstriction. The results were as shown in Fig. 6.

While the present inventors had previously reported that chemically synthesized [Leu¹⁷]-VIP-Hse is at least three times more active than native VIP in a test using the guinea pig bronchial smooth muscle (JP-A-4-230400), the present test supported this fact.

### PRODUCTION EXAMPLE 2

This example illustrates the production of a polypeptide hereinafter referred to as VIP#10-Hse (SEQ ID NO: 30) using Sarcophaga lectin as a leader peptide (Hse being encoded as methionine in DNA).

VIP#10-Hse (SEQ ID NO: 30)

His-Ser-Asp-Ala-Val-Phe-Thr-Gly-Asn-Tyr-Thr-Lys-Leu-Arg-Lys-

Gln-Leu-Ala-Ala-Lys-Lys-Tyr-Leu-Asn-Lys-Ala-Leu-Lys-Hse

### a) Step 1: Synthesis of a VIP#10-Hse gene

In the above polypeptide, the Hse residue at position 29 is derived from methionine by treatment with cyanogen bromide following polypeptide production in Escherichia coli. Therefore, a double-stranded DNA (SEQ ID NOs: 31 and 32) fragment having a base sequence coding for the above amino acid sequence with methionine at position 29 in lieu of Hse, namely a double-stranded DNA fragment of the following formula (12) (SEQ ID NOs: 31 and 32), was prepared. 
Formula (12) (SEQ ID NOs: 31 and 32)

  1        10        20        30        40        50        60
5'-GATCTTCATGCATTCTGATGCTGTATTCACTGGTAACTACACTAAACTGCGTAAACAGCT
3'-    AAGTACGTAAGACTACGACATAAGTGACCATTGATGTGATTTGACGCATTTGTCGA

          70        80        90        100       110
   GGCTGCTAAGAAATACCTGAACAAGGCTCTGAAGATGGGGATCCTGTGATA    -3'
   CCGACGATTCTTTATGGACTTGTTCCGAGACTTCTACCCCTAGGACACTATTCGA -5'
The double-stranded DNA of the above formula (12) (SEQ ID NOs: 31 and 32) was constructed in the following manner.

Single-stranded DNAs respectively having the base sequences shown below in terms of formulas (13) to (20) (SEQ ID NOs: 33 to 40) were synthesized using a DNA synthesizer (Pharmacia's Gene Assembler). As is evident from these formulas, (13) (SEQ ID NO: 33) and (17) (SEQ ID NO: 37), (14) (SEQ ID NO: 34) and (18) (SEQ ID NO: 38), (15) (SEQ ID NO: 35) and (19) (SEQ ID NO: 39), and (16) (SEQ ID NO: 36) and (20) (SEQ ID NO: 40) are complementary to each other except for terminal portions and each pair can form a double-stranded DNA.
Formula (13) (SEQ ID NO: 33)
5'-GATCTTCATGCATTCTGATGCTGTATT-3'
Formula (14) (SEQ ID NO: 34)
5'-CACTGGTAACTACACTAAACTGCGTAAA-3'
Formula (15) (SEQ ID NO: 35)
5'-CAGCTGGCTGCTAAGAAATACCTGAACA-3'
Formula (16) (SEQ ID NO: 36)
5'-AGGCTCTGAAGATGGGGATCCTGTGATA-3'
Formula (17) (SEQ ID NO: 37)
5'-CCAGTGAATACAGCATCAGAATGCATGAA-3'
Formula (18) (SEQ ID NO: 38)
5'-CCAGCTGTTTACGCAGTTTAGTGTAGTTA-3'
Formula (19) (SEQ ID NO: 39)
5'-GAGCCTTGTTCAGGTATTTCTTAGCAG-3'
Formula (20) (SEQ ID NO: 40)
5'-AGCTTATCACAGGATCCCCATCTTCA-3'
The above single-stranded DNAs were respectively 5'-phosphorylated by treatment with polynucleotide kinase and then the pairs of complementary DNAs, namely (13) (SEQ ID NO: 33) and (17) (SEQ ID NO: 37), (14) (SEQ ID NO: 34) and (18) (SEQ ID NO: 38), (15) (SEQ ID NO: 35) and (19) (SEQ ID NO: 39), and (16) (SEQ ID NO: 16) and (20) (SEQ ID NO: 40), were subjected to annealing. The resulting four double-stranded DNAs were ligated together in tandem using DNA ligase to give the double-stranded DNA fragment of formula (12) (SEQ ID NOs: 31 and 32). This synthetic gene codes for VIP#10-Met-Gly-Ile-Leu following Ile-Phe-Met (SEQ ID NO: 41). Since, however, as shown in the steps mentioned below, cyanogen bromide treatment following fused protein expression in Escherichia coli results in cleavage of methionine on the C-terminal side, giving homoserine (inclusive of homoserine lactone), VIP#10-Hse (SEQ ID NO: 30) can eventually be obtained. The amino acid sequence of the portion for connecting VIP#10-Hse is not limited to the amino acid sequence shown above provided that the C terminus is methionine. This is because methionine is degraded by cyanogen bromide treatment in the process of purification and monomeric VIP#10-Hse (SEQ ID NO: 30) is released.

### b) Step 2: Insertion of the VIP#10-Met-encoding DNA into a vector and pluralization of the VIP#10-Met gene portion

In the following, a procedure for inserting the synthetic DNA fragment (SEQ ID NOs: 31 and 32) obtained in Example 2, Step 1 into an expression vector is illustrated.

The plasmid (pTMH4) described in Example 1, Step 2 was cleaved with the restriction enzymes BglII and HindIII and a DNA fragment of about 4.7 Kbp was purified. This was ligated to the DNA fragment of formula (12) (SEQ ID NOs: 31 and 32) for reconstruction. The resulting plasmid is herein-after referred to as pQ6V10H1. Then, pQ6V10H1 was cleaved with PstI and BglII and a VIP#10-Met gene-containing DNA fragment was purified (DNA 2-I). Separately, pQ6V10H1 was cleaved with PstI and BamHI and a VIP#10-Met gene-containing DNA fragment was purified (DNA 2-II). DNA 2-I was ligated to DNA 2-II for reconstruction, whereby a plasmid, pQ6V10H2, containing two molecules of the VIP#10-Met gene connected in tandem was obtained. On that occasion, the BglII cleavage site and the BamHI cleavage site are ligated together by means of DNA ligase and the ligation site is no longer a recognition site for either restriction enzyme. Therefore, a plasmid containing any desired number of VIP#10-Met genes connected in tandem can be obtained by repeating the above-mentioned cleavage and ligation procedures. In this manner, the present inventors prepared plasmids containing 3 to 15 VIP#10-Met genes connected in tandem, respectively named pQ6V10H3, pQ6V10H4, PQ6V10H5, pQ6V10H6, pQ6V10H7, PQ6V10H8, PQ6V10H9, pQ6V10H10, pQ6V10H11, pQ6V10H12, pQ6V10H13, pQ6V10H14, and pQ6V10H15.

### c) Step 3: Insertion of a Sarcophaga lectin (leader peptide) gene into the vectors

A procedure for introducing a gene for Sarcophaga lectin as a leader peptide into the plasmids obtained in Example 2, Step 2 is described in the following.

A plasmid (pLTA160) for use in the expression of Sarcophaga lectin (Journal of Biological Chemistry, vol. 260, pages 12228-12233, 1985) was cleaved with the restriction enzymes EcoT22I and ScaI and a DNA fragment containing the gene for an N-terminal portion of Sarcophaga lectin was isolated. This DNA fragment was rendered blunt-ended using a Takara Shuzo blunting kit (the resulting DNA fragment is hereinafter referred to as DNA 2-III). Separately, pQ6V10H1 was cleaved with the restriction enzymes ScaI and BglII, followed by isolation and the same blunting treatment as mentioned above (DNA 2-IV). DNA 2-III and DNA 2-IV were subjected to double-stranded DNA ligation using DNA ligase. Thus was constructed a plasmid, pTLV10H1, coding for a fused polypeptide comprising a polypeptide composed of the 24th to 265th amino acid residues of Sarcophaga lectin and one VIP#10-Met molecule coupled thereto. By treating the plasmids pQ6V10H2, pQ6V10H3, pQ6V10H4, pQ6V10H5, pQ6V10H6, pQ6V10H7, pQ6V10H8, pQ6V10H9, pQ6V10H10, pQ6V10H11, pQ6V10H12, pQ6V10H13, pQ6V10H14, and pQ6V10H15 respectively in the same manner, namely subjecting them to cleavage with the restriction enzymes ScaI and BglII, followed by isolation, blunting treatment and ligation of the respective blunt-ended DNA fragments to DNA 2-III, the present inventors further prepared plasmids containing a gene coding for a polypeptide composed of the 24th to 265th amino acid residues of Sarcophage lectin and 2 to 15 VIP#10-Met molecules tandem-connected and coupled to said polypeptide, namely the plasmids pTLV10H2, pTLV10H3, pTLV10H4, pTLV10H5, pTLV10H6, pTLV10H7, pTLV10H8, pTLV10H9, pTLV10H10, pTLV10H11, pTLV10H12, pTLV10H13, pTLV10H14 and pTLV10H15.

### d) Step 4: Construction of plasmids for expression of the fused proteins

A procedure for constructing plasmids for fused protein expression is now described.

The plasmid pKK223-3 (Pharmacia) was cleaved with the restriction enzymes EcoRI and HindIII, followed by isolation (DNA 2-V). Separately, pTLV10H1 was cleaved with the restriction enzymes EcoRI and HindIII, followed by isolation and ligation to DNA 2-V, whereby an expression plasmid, pILV10H1, containing the tac promoter and a succeeding gene coding for a fused protein composed of a polypeptide comprising the 24th to 265th amino acid residues of Sarcophaga lectin and one VIP#10-Met molecule coupled to said polypeptide was obtained. By treating the plasmids pTLV10H2, pTLV10H3, pTLV10H4, pTLV10H5, pTLV10H6, pTLV10H7, pTLV10H8, pTLV10H9, pTLV10H10, pTLV10H11, pTLV10H12, pTLV10H13, pTLV10H14, and pTLV10H15, respectively in the same manner, namely subjecting them to cleavage with the restriction enzymes EcoRI and HindIII, followed by isolation and ligation to DNA 2-V, there were obtained plasmids, pILV10H2, pILV10H3, pILV10H4, pILV10H5, pILV10H6, pILV10H7, pILV10H8, pILV10H9, pILV10H10, pILV10H11, pILV10H12, pILV10H13, pILV10H14, and pILV10H15, containing the tac promoter and a subsequent gene coding for a polypeptide composed of the 24th to 265th amino acid residues of Sarcophaga lectin and 2 to 15 VIP#10-Met molecules tandem-connected and, as a whole, coupled to said polypeptide.

### e) Step 5: Paring of Sarcophaga lectin as the leader peptide

For commercial use of the VIP#10-Hse (SEQ ID NO: 30), it is desirable that the proportion of the VIP#10-Hse (SEQ ID NO: 30) in the fused protein expressed be increased and that plasmids of high-level expression be selected. Therefore, mention is now made of a procedure for shortening the Sarcophaga lectin portion to serve as the leader peptide by using the plasmids obtained in Example 1, Step 5.

The plasmid pIL162VH1 described in Example 1, Step 5 was cleaved with the restriction enzymes EcoRI and BglII and a DNA containing a gene coding for the 24th to 59th amino acid residues of Sarcophaga lectin was isolated (DNA 2-VI). Separately, pILV10H1 was cleaved with the restriction enzymes EcoRI and BglII and a VIP#10-Met gene-containing DNA was isolated and ligated to DNA 2-VI to give an expression plasmid, pIL162V10H1, coding for a fused protein composed of a polypeptide comprising the 24th to 59th amino acid residues of Sarcophaga lectin and one VIP#10-Met molecule coupled thereto. By treating the plasmids pILV10H2, pILV10H3, pILV10H4, pILV10H5, pILV10H6, pILV10H7, pILV10H8, pILV10H9, pILV10H10, pILV10H11, pILV10H12, pILV10H13, pILV10H14 and pILV10H15 respectively in the same manner, namely subjecting them to cleavage with the restriction enzymes EcoRI and BglII, followed by isolation of a VIP#10-Met gene-containing DNA and ligation thereof to DNA 2-VI, there were obtained plasmids, pIL162V10H2, pIL162V10H3, pIL162V10H4, pIL162V10H5, pIL162V10H6, pIL162V10H7, pIL16210H8, pIL162V10H9, pIL162V10H10, pIL162V10H11, pIL162V10H12, pIL162V10H13, pIL162V10H14 and pIL162V10H15, containing the tac promoter and a succeeding gene coding for a polypeptide composed of the 24th, 59th amino acid residues of Sarcophaga lectin and 2 to 15 VIP#10-Met molecules tandem-connected and, as a whole, coupled to said polypeptide.

Then, pIL288VH1 was cleaved with the restriction enzymes EcoRI and BglII and DNA containing a gene for the 24th to 101st amino acid residues of Sarcophaga lectin was isolated (DNA 2-VII). Separately, pILV10H1 was cleaved with the restriction enzymes EcoRI and BglII and DNA containing a gene for VIP#10-Met was isolated and ligated to DNA 2-VII to give an expression plasmid, pIL288V10H1, containing the tac promoter and a succeeding gene coding for a fused protein consisting of a polypeptide composed of the 24th to 101st amino acid residues of Sarcophaga lectin and one VIP#10-Met molecule coupled thereto. By treating the plasmids pILV10H2, pILV10H3, pILV10H4, pILV10H5, pILV10H6, pILV10H7, pILV10H8, pILV10H9, pILV10H10, pILV10H11, pILV10H12, pILV10H13, pILV10H14 and pILV10H15 respectively in the same manner, namely subjecting them to cleavage with the restriction enzymes EcoRI and BglII, followed by isolation of a VIP#10-Met gene-containing DNA and ligation thereof to DNA 2-VII, there were obtained plasmids, pIL288V10H2, pIL288V10H3, pIL28810H4, pIL288V10H5, pIL288V10H6, pIL288V10H7, pIL288V10H8, pIL288V10H9, pIL288V10H10, pIL288V10H11, pIL288V10H12, pIL288V10H13, pIL288V10H14 and pIL288V10H15, containing the tac promoter and a succeeding gene coding for a polypeptide composed of the 24th to 101st amino acid residues of Sarcophaga lectin and 2 to 15 VIP#10-Met molecules tandem-connected and, as a whole, coupled to said polypeptide.

Then, pIL496VH1 was cleaved with the restriction enzymes EcoRI and BglII and DNA containing a gene for the 24th to 171st amino acid residues of Sarcophaga lectin was isolated (DNA 2-VIII). Separately, pILV10H1 was cleaved with the restriction enzymes EcoRI and BglII and a VIP#10-Met gene-containing DNA was isolated and ligated to DNA 2-VIII to give an expression plasmid, pIL496V10H1, containing the tac promoter and a succeeding gene coding for a fused protein consisting of a polypeptide composed of the 24th to 171st amino acid residues of Sarcophaga lectin and one VIP#10-Met molecule coupled thereto. By treating the plasmids pILV10H2, pILV10H3, pILV10H4, pILV10H5, pILV10H6, pILV10H7, pILV10H8, pILV10H9, pILV10H10, pILV10H11, pILV10H12, pILV10H13, pILV10H14 and pILV10H15 respectively in the same manner, namely subjecting them to cleavage with the restriction enzymes EcoRI and BglII, followed by isolation of a VIP#10-Met gene-containing DNA and ligation thereof to DNA 2-VIII, there were obtained plasmids, pIL496V10H2, pIL496V10H3, pIL496V10H4, pIL496V10H5, pIL496V10H6, pIL496V10H7, pIL496V10H8, pIL496V10H9, pIL496V10H10, pIL496V10H11, pIL496V10H12, pIL496V10H13, pIL496V10H14 and pIL496V10H15, containing the tac promoter and a succeeding gene coding for a polypeptide composed of the 24th to 171st amino acid residues of Sarcophaga lectin and 2 to 15 VIP#10-Met molecules tandem-connected and, as a whole, coupled to said polypeptide.

Then, pIL135VH1 was cleaved with the restriction enzymes EcoRI and BglII and a DNA containing a gene coding for the 24th to 50th amino acid residues of Sarcophaga lectin was isolated (DNA 2-IX). Separately, pILV10H1 was cleaved with the restriction enzymes EcoRI and BglII and a VIP#10-Met gene-containing DNA was isolated and ligated to DNA 2-IX to give an expression plasmid, pIL135V10H1, containing the tac promoter and a succeeding gene coding for a fused protein consisting of a polypeptide composed of the 24th to 50th amino acid residues of Sarcophaga lectin and one VIP#10-Met molecule coupled thereto. By treating the plasmids pILV10H2, pILV10H3, pILV10H4, pILV10H5, pILV10H6, pILV10H7, pILV10H8, pILV10H9, pILV10H10, pILV10H11, pILV10H12, pILV10H13, pILV10H14 and pILV10H15 respectively in the same manner, namely subjecting them to cleavage with the restriction enzymes EcoRI and BglII, followed by isolation of a VIP#10-Met gene-containing DNA and ligation thereof to DNA 2-IX, there were obtained plasmids, pIL135V10H2, pIL135V10H3, pIL135V10H4, pIL135V10H5, pIL135V10H6, pIL135V10H7, pIL135V10H8, pIL135V10H9, pIL135V10H10, pIL135V10H11, pIL135V10H12, pIL135V10H13, pIL135V10H14 and pIL135V10H15, containing the tac promoter and a succeeding gene coding for a polypeptide composed of the 24th to 50th amino acid residues of Sarcophaga lectin and 2 to 15 VIP#10-Met molecules tandem-connected and, as a whole, coupled to said polypeptide.

### f) Step 6: Production of fused proteins

Escherichia coli JM109 was transformed with the fused protein expression plasmids obtained in Example 2, Step 4 and Step 5, namely pILV10H1, pILV10H2, pILV10H3, pILV10H4, pILV10H5, pILV10H6, pILV10H7, pILV10H8, pILV10H9, pILV10H10, pILV10H11, pILV10H12, pILV10H13, pILV10H14, pILV10H15, pIL135V10H1, pIL135V10H2, pIL135V10H3, pIL135V10H4, pIL135V10H5, pIL135V10H6, pIL135V10H7, pIL135V10H8, pIL135V10H9, pIL135V10H10, pIL135V10H11, pIL135V10H12, pIL135V10H13, pIL135V10H14, pIL135V10H15, pIL162V10H1, pIL162V10H2, pIL162V10H3, pIL162V10H4, pIL162V10H5, pIL162V10H6, pIL162V10H7, pIL162V10H8, pIL162V10H9, pIL162V10H10, pIL162V10H11, pIL162V10H12, pIL162V10H13, pIL162V10H14, pIL162V10H15, pIL288V10H1, pIL288V10H2, pIL288V10H3, pIL288V10H4, pIL288V10H5, pIL288V10H6, pIL288V10H7, pIL288V10H8, pIL288V10H9, pIL288V10H10, pIL288V10H11, pIL288V10H12, pIL288V10H13, pIL288V10H14, pIL288V10H15, pIL496V10H1, pIL496V10H2, pIL496V10H3, pIL496V10H4, pIL496V10H5, pIL496V10H6, pIL496V10H7, pIL496V10H8, pIL496V10H9, pIL496V10H10, pIL496V10H11, pIL496V10H12, pIL496V10H13, pIL496V10H14, and pIL496V10H15, respectively. The transformants were respectively cultured in a medium (e.g. L medium, amino acid-containing M9 medium) containing 50 µg/ml of ampicillin under aeration at a temperature of 25° to 40°C, preferably 30° to 38°C using an Abel type D fermenter. When the A₅₅₀ value reached 0.6 to 0.8, isopropyl-1-thio-β-D-galactoside (IPTG) was added to a final concentration of 1 mM.

Cultivation was further continued for 5 to 24 hours and then cells were recovered by centrifugation (5,000 rpm, 4°C, 20 minutes). Each lot of cells was sampled and analyzed by 12.5% SDS polyacrylamide gel electrophoresis, whereupon a band was detected at a position corresponding to the molecular weight of the desired fused protein. The bands thus detected were measured using a densitometer, giving the data shown in Table 2.

### g) Step 7: Isolation/purification and identification of VIP#10-Hse

The Escherichia coli cell sediment obtained by using pIL496V10H7 for transformation was treated by the procedure described in Example 1, Step 7, for purification of VIP#10-Hse. The purified product was submitted to an ABI peptide sequencer, which proved conclusively that VIP#10-Hse had been purified.

The above procedure gave 100 mg of VIP#10-Hse per liter of Escherichia coli culture.

**TABLE 2**

| Plasmid | Fused protein in cell protein (%) | Plasmid | Fused protein in cell protein (%) | Plasmid | Fused protein in cell protein (%) |
|---|---|---|---|---|---|
| pILV10H1 | <2 | pIL135V10H11 | 9 | pIL288V10H6 | 22 |
| pILV10H2 | <2 | pIL135V10H12 | 5 | pIL288V10H7 | 27 |
| pILV10H3 | <2 | pIL135V10H13 | <2 | pIL288V10H8 | 20 |
| pILV10H4 | 13 | pIL135V10H14 | <2 | pIL288V10H9 | 17 |
| pILV10H5 | 5 | pIL135V10H15 | <2 | pIL288V10H10 | 10 |
| pILV10H6 | 15 | pIL162V10H1 | <2 | pIL288V10H11 | <2 |
| pILV10H7 | 15 | pIL162V10H2 | <2 | pIL288V10H12 | <2 |
| pILV10H8 | 12 | pIL162V10H3 | <2 | pIL288V10H13 | <2 |
| pILV10H9 | 10 | pIL162V10H4 | 10 | pIL288V10H14 | <2 |
| pILV10H10 | 10 | pIL162V10H5 | 7 | pIL288V10H15 | <2 |
| pILV10H11 | 8 | pIL162V10H6 | 20 | pIL496V10H1 | <2 |
| pILV10H12 | 5 | pIL162V10H7 | 25 | pIL496V10H2 | <2 |
| pILV10H13 | <2 | pIL162V10H8 | 18 | pIL496V10H3 | <2 |
| pILV10H14 | <2 | pIL162V10H9 | 15 | pIL496V10H4 | 20 |
| pILV10H15 | <2 | pIL162V10H10 | 8 | pIL496V10H5 | 10 |
| pIL135V10H1 | <2 | pIL162V10H11 | <2 | pIL496V10H6 | 26 |
| pIL135V10H2 | <2 | pIL162V10H12 | <2 | pIL496V10H7 | 30 |
| pIL135V10H3 | <2 | pIL162V10H13 | <2 | pIL496V10H8 | 27 |
| pIL135V10H4 | 15 | pIL162V10H14 | <2 | pIL496V10H9 | 15 |
| pIL135V10H5 | 7 | pIL162V10H15 | <2 | pIL496V10H10 | 11 |
| pIL135V10H6 | 17 | pIL288V10H1 | <2 | pIL496V10H11 | 11 |
| pIL135V10H7 | 17 | pIL288V10H2 | <2 | pIL496V10H12 | <2 |
| pIL135V10H8 | 13 | pIL288V10H3 | <2 | pIL496V10H13 | <2 |
| pIL135V10H9 | 11 | pIL288V10H4 | 13 | pIL496V10H14 | <2 |
| pIL135V10H10 | 10 | pIL288V10H5 | 9 | pIL496V10H15 | <2 |

### PHYSIOLOGICAL ACTIVITY TEST EXAMPLE 2

VIP#10-Hse obtained in the above manner was tested for the dose required for inhibiting histamine-induced airway contraction by 50% in guinea pigs by the Konzett-Roessler method, with native VIP as a control. Whereas said dose was 7.5 µg/kg for native VIP, that for VIP#10-Hse was 0.4 µg/kg. The latter value was in good agreement with the value for chemically synthesized VIP#10-Hse (JP-A-4-230400).

In the Production Example 1, when the recombinant plasmids pIL135VH1 to pIL135VH10 containing a gene sequence for the 24th to 50th amino acid residues of Sarcophaga lectin were used, the resulting Escherichia coli transformants failed to express the desired fused proteins. However, when the recombinant plasmids pIL162VH1 to pIL162VH10 containing a gene sequence for the polypeptide covering the 24th to 59th amino acid residues of Sarcophaga lectin as a leader peptide were used for transformation of Escherichia coli, fused protein expression was observed with the transformants. It is therefore estimated that the molecular weight of the leader peptide is preferably not less than 5,000. Furthermore, it can be seen from Table 1 that the use of expression plasmids containing 4 molecules of the [Leu¹⁷]-VIP-Met gene arranged in tandem is particularly advantageous.

In the Production Example 2, on the other hand, when the recombinant plasmids pIL135V10H1 to pIL135V10H15 containing a gene sequence for the 24th to 50th amino acid residues of Sarcophaga lectin were used, the desired fused protein expression was observed. Furthermore, it can be seen from Table 2 that the plasmids containing 4 to 10 molecules of VIP#10-Met gene arranged in tandem is preferred.

As described above, the present invention makes it possible to produce [Leu¹⁷]-VIP-Hse and VIP#10-Hse, which are several times more active than native VIP and is highly stable, with ease and in large quantities by using Escherichia coli transformants. This means that VIP and VIP analogs, which have been expected to be useful in the treatment of bronchial asthma and impotence but cannot have been produced in large quantities, can now be supplied, in the form of VIP analogs having the above structure, to the market in large quantities and at low cost.

While the invention has been described in detail and with reference to specific embodiments thereof, it will be apparent to one skilled in the art that various changes and modifications can be made therein without departing from the spirit and scope thereof.

## Claims

1. A fused protein for VIP analog production which comprises a leader peptide derived from Sarcophaga lectin, and one molecule, or a plurality of molecules arranged in tandem, of a peptide of the general formula (1) (SEQ ID NO: 1): 
His-Ser-Asp-Ala-Val-Phe-Thr-R1-R2-Tyr-Thr-R3-Leu-Arg-Lys-Gln-
Leu-Ala-R4-R5-Lys-Tyr-Leu-R6-R7-R8-R9-R10-Met
 wherein R₁ is Asp or Gly, R₂ is Asn or Gln, R₃ is Arg or Lys, R₄ is Val, Ala or Lys, R₅ is Lys or Leu, R₆ is Asn, Gln or Lys, R₇ is Ser, Lys or Ala, R₈ is Ile, Ala or Leu, R₉ is Leu or Lys and R₁₀ is Asn, Lys or Arg, linked to said leader peptide in a downstream direction.

2. The fused protein for VIP analog production as claimed in Claim 1, wherein, in general formula (1) (SEQ ID NO: 1), R₁ is Gly, R₂ is Asn, R₃ is Lys, R₄ is Ala, R₅ is Lys, R₆ is Asn or Lys, R₇ is Lys or Ala, R₈ is Ala or Leu, R₉ is Leu or Lys and R₁₀ is Lys or Arg.

3. The fused protein for VIP analog production as claimed in Claim 1 or 2, wherein leader peptide derived from Sarcophaga lectin has a molecular weight of 5000 or more.

4. The fused protein for VIP analog production as claimed in Claim 1 or 2, wherein four to ten molecules of the peptide of general formula (1) (SEQ ID NO: 1) are linked in tandem.

5. A method of producing VIP analogs of the general formula (2) (SEQ ID NO: 2): 
His-Ser-Asp-Ala-Val-Phe-Thr-R1-R2-Tyr-Thr-R3-Leu-Arg-Lys-Gln-
Leu-Ala-R4-R5-Lys-Tyr-Leu-R6-R7-R8-R9-R10-Hse
 wherein R₁ is Asp or Gly, R₂ is Asn or Gln, R₃ is Arg or Lys, R₄ is Val, Ala or Lys, R₅ is Lys or Leu, R₆ is Asn, Gln or Lys, R₇ is Ser, Lys or Ala, R₈ is Ile, Ala or Leu, R₉ is Leu or Lys, R₁₀ is Asn, Lys or Arg and Hse is a homoserine or homoserine lactone residue, which comprises treating the fused protein of Claim 1 with cyanogen bromide.

6. A recombinant plasmid which contains a DNA coding for a polypeptide comprising a leader peptide derived from Sarcophaga lectin, and one molecule, or a plurality of molecules arranged in tandem, of a peptide of the general formula (1) (SEQ ID NO: 1): 
His-Ser-Asp-Ala-Val-Phe-Thr-R1-R2-Tyr-Thr-R3-Leu-Arg-Lys-Gln-
Leu-Ala-R4-R5-Lys-Tyr-Leu-R6-R7-R8-R9-R10-Met
 wherein R₁ is Asp or Gly, R₂ is Asn or Gln, R₃ is Arg or Lys, R₄ is Val, Ala or Lys, R₅ is Lys or Leu, R₆ is Asn, Gln or Lys, R₇ is Ser, Lys or Ala, R₈ is Ile, Ala or Leu, R₉ is Leu or Lys and R₁₀ is Asn, Lys or Arg, linked to said leader peptide in a downstream direction.

7. The recombinant plasmid as claimed in Claim 6, wherein, in general formula (1) (SEQ ID NO: 1), R₁ is Gly, R₂ is Asn, R₃ is Lys, R₄ is Ala, R₅ is Lys, R₆ is Asn or Lys, R₇ is Lys or Ala, R₈ is Ala or Leu, R₉ is Leu or Lys and R₁₀ is Lys or Arg.

8. The recombinant plasmid as claimed in Claim 6 or 7, wherein leader peptide derived from Sarcophaga lectin having a molecular weight of 5000 or more.

9. The transformant microorganism carrying the plasmid of Claim 6 or 7.

10. The transformant microorganism of Claim 7, wherein the microorganism transformed is Escherichia coli strain JM109 or HB101.


SEQUENCE LISTING

(1) GENERAL INFORMATION:

(i) APPLICANT:
(A) NAME: SANWA KAGAKU KENKYUSHO CO., LTD.
(B) STREET: 35, Higashisotobori-cho, Higashi-ku
(C) CITY: Nagoya
(D) STATE: Aichi
(E) COUNTRY: Japan
(F) POSTAL CODE (ZIP): 461
(G) TELEPHONE: 052-951-8130
(H) TELEFAX: 052-953-1241

(ii) TITLE OF INVENTION: FUSED PROTEINS FOR PREPARING VASOACTIVE
INTESTINAL POLYPEPTIDE ANALOGS, METHOD OF PREPARING SAME
AND RECOMBINANT PLASMIDS AND TRANSFORMANT MICROORGANISMS

(iii) NUMBER OF SEQUENCES: 41

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: JP Hei. 5-099313
(B) FILING DATE: 26-APR-1993

(2) INFORMATION FOR SEQ ID NO:1:

(i) SEQUENCE CHARACTERISTICS:
(A) LENGTH: 29 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:1:

His Ser Asp Ala Val Phe Thr Xaa Xaa Tyr Thr Xaa Leu Arg Lys Gln
1               5                   10                  15

Leu Ala Xaa Xaa Lys Tyr Leu Xaa Xaa Xaa Xaa Xaa Met
            20                  25

(2) INFORMATION FOR SEQ ID NO:2:

(i) SEQUENCE CHARACTERISTICS:
(A) LENGTH: 29 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:2:

His Ser Asp Ala Val Phe Thr Xaa Xaa Tyr Thr Xaa Leu Arg Lys Gln
1               5                   10                  15

Leu Ala Xaa Xaa Lys Tyr Leu Xaa Xaa Xaa Xaa Xaa Xaa
            20                  25

(2) INFORMATION FOR SEQ ID NO:3:

(i) SEQUENCE CHARACTERISTICS:
(A) LENGTH: 29 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:3:

His Ser Asp Ala Val Phe Thr Asp Asn Tyr Thr Arg Leu Arg Lys Gln
1               5                   10                  15

Leu Ala Val Lys Lys Tyr Leu Asn Ser Ile Leu Asn Met
            20                  25

(2) INFORMATION FOR SEQ ID NO:4:

(i) SEQUENCE CHARACTERISTICS:
(A) LENGTH: 111 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:4:

GATCTTCATG CATTCAGATG CAGTGTTCAC TGACAACTAC ACCCGCCTGC GCAAACAGCT 60

GGCTGTAAAG AAATACCTGA ACTCAATATT GAACATGGGG ATCCTGTGAT A 111

(2) INFORMATION FOR SEQ ID NO:5:

(i) SEQUENCE CHARACTERISTICS:
(A) LENGTH: 111 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:5:

AGCTTATCAC AGGATCCCCA TGTTCAATAT TGAGTTCAGG TATTTCTTTA CAGCCAGCTG 60

TTTGCGCAGG CGGGTGTAGT TGTCAGTGAA CACTGCATCT GAATGCATGA A 111

(2) INFORMATION FOR SEQ ID NO:6:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:6:

GATCTTCATG CATTCAGATG CAGTGTT 27

(2) INFORMATION FOR SEQ ID NO:7:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:7:

CACTGACAAC TACACCCGCC TGCGCAAA 28

(2) INFORMATION FOR SEQ ID NO:8:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:8:

CAGCTGGCTG TAAAGAAATA CCTGAACT 28

(2) INFORMATION FOR SEQ ID NO:9:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:9:

CAATATTGAA CATGGGGATC CTGTGATA 28

(2) INFORMATION FOR SEQ ID NO:10:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:10:

TCAGTGAACA CTGCATCTGA ATGCATGAA 29

(2) INFORMATION FOR SEQ ID NO:11:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:11:

CCAGCTGTTT GCGCAGGCGG GTGTAGTTG 29

(2) INFORMATION FOR SEQ ID NO:12:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:12:

ATATTGAGTT CAGGTATTTC TTTACAG 27

(2) INFORMATION FOR SEQ ID NO:13:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:13:

AGCTTATCAC AGGATCCCCA TGTTCA 26

(2) INFORMATION FOR SEQ ID NO:14:

(i) SEQUENCE CHARACTERISTICS:

(A) LENGTH: 35 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:14:

Ile Phe Met His Ser Asp Ala Val Phe Thr Asp Asn Tyr Thr Arg Leu
1               5                   10                  15

Arg Lys Gln Leu Ala Val Lys Lys Tyr Leu Asn Ser Ile Leu Asn Met
            20                  25                  30

Gly Ile Leu
        35

(2) INFORMATION FOR SEQ ID NO:15:

(i) SEQUENCE CHARACTERISTICS:
(A) LENGTH: 108 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:15:

AATTAATTCC ATTCAACGCC AGTCCCGAAC GTGAAATTTC CTCTCTTGCT GGCGCGATTG 60

CAGCTGTGGT GTCATGGTCG GTGATCGCCA GGGTGCCGAC GCGCATCT 108

(2) INFORMATION FOR SEQ ID NO:16:

(i) SEQUENCE CHARACTERISTICS:
(A) LENGTH: 106 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:16:

CGAGATGCGC GTCGGCACCC TGGCGATCAC CGACCATGAC ACCACAGCTG CAATCGCGCC 60

AGCAAGAGAG GAAATTTCAC GTTCGGGACT GGCGTTGAAT GGAATT 106

(2) INFORMATION FOR SEQ ID NO:17:

(i) SEQUENCE CHARACTERISTICS:
(A) LENGTH: 55 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:17:

TAATCATCGA ACTAGTTAAC TAGTACGCAA TAACTCAATC TAGAACGGAG GTTTG 55

(2) INFORMATION FOR SEQ ID NO:18:

(i) SEQUENCE CHARACTERISTICS:
(A) LENGTH: 57 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:18:

AATTCAAACC TCCGTTCTAG ATTGAGTTAT TGCGTACTAG TTAACTAGTT CGATGAT 57

(2) INFORMATION FOR SEQ ID NO:19:

(i) SEQUENCE CHARACTERISTICS:
(A) LENGTH: 346 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:19:

AATTAATTCC ATTCAACGCC AGTCCCGAAC GTGAAATTTC CTCTCTTGCT GGCGCGATTG 60

CAGCTGTGGT GTCATGGTCG GTGATCGCCA GGGTGCCGAC GCGCATCTCG ACTGCACGGT 120

GCACCAATGC TTCTGGCGTC AGGCAGCCAT CGGAAGCTGT GGTATGGCTG TGCAGGTCGT 180

AAATCACTGC ATAATTCGTG TCGCTCAAGG CGCACTCCCG TTCTGGATAA TGTTTTTTGC 240

GCCGACATCA TAACGGTTCT GGCAAATATT CTGAAATGAG CTGTTGACAA TTAATCATCG 300

AACTAGTTAA CTAGTACGCA ATAACTCAAT CTAGAACGGA GGTTTG 346

(2) INFORMATION FOR SEQ ID NO:20:

(i) SEQUENCE CHARACTERISTICS:
(A) LENGTH: 346 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:20:

AATTCAAACC TCCGTTCTAG ATTGAGTTAT TGCGTACTAG TTAACTAGTT CGATGATTAA 60

TTGTCAACAG CTCATTTCAG AATATTTGCC AGAACCGTTA TGATGTCGGC GCAAAAAACA 120

TTATCCAGAA CGGGAGTGCG CCTTGAGCGA CACGAATTAT GCAGTGATTT ACGACCTGCA 180

CAGCCATACC ACAGCTTCCG ATGGCTGCCT GACGCCAGAA GCATTGGTGC ACCGTGCAGT 240

CGAGATGCGC GTCGGCACCC TGGCGATCAC CGACCATGAC ACCACAGCTG CAATCGCGCC 300

AGCAAGAGAG GAAATTTCAC GTTCGGGACT GGCGTTGAAT GGAATT 346

(2) INFORMATION FOR SEQ ID NO:21:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:21:

TTGAATTCAT GGTGCCCCAA TTACAAAAGG CT 32

(2) INFORMATION FOR SEQ ID NO:22:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:22:

GAAGCTTTTA ACAAATTTCA TTATTTTCTG C 31

(2) INFORMATION FOR SEQ ID NO:23:

(i) SEQUENCE CHARACTERISTICS:
(A) LENGTH: 792 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:23:

AATTCATGGT GCCCCAATTA CAAAAGGCTT TAGATGGCAG AGAATATCTT ATTGAAACAG 60

AACTTAAGTA CAATTGGCAT CAAGCCTGGC ATGAATGTGC CCGCCATGAT CAACAACTTG 120

TAACAATCGA AAGTGCTGAT AAGAATAATG CCATTATCGA TCTGGTGAAA CGGGTCGTTG 180

GAAAATCTCA TAATTTATGG TTGGGCGGCA ATGATGAGTA TAGTTCAAGT CGTGACTATG 240

GCAGACCCTT TTTCTGGTCA CCCACCGGTC AAGCATTCTC CTTTGCCTAC TGGTCGGAAA 300

ACAATCCCGA TAATTATAAG CATCAAGAAC ATTGTGTCCA TATATGGGAT ACAAAGCCCT 360

TATATCAATG GAACGATAAT GATTGTAATG TTAAAATGGG TTACATATGT GAACCCAATC 420

ATTTCCGGGA AACATATGAT CAAGCACTCA AGCAAAAATG CGAAGCAATT AAGATAACAA 480

ATTCAAAAAT TTCAACAGAA TTTGATCAAT TGCATGCCAA ACAATCATTG GAATTTGATA 540

GTATAACGCA AAATGTAGCA AAAGTGAATG AAGATTGGAA AATTGAAATC CAAAAACTAC 600

AGAATGCCAC ACAAATTGCC ATACAACAGA TTATGGAAAA TCATGAGAAG AAGATAAGAG 660

ATTTAAGTGA TAATCTACTT AAGCAGCTAC AAGATTCCAA TGAACAACTG AAACAGTCCA 720

CTGACCATAT GAATGCATCG TTTGGTGAGA AATTGAAAGG CCAACAAGCA GAAAATAATG 780

AAATTTGTTA AA 792

(2) INFORMATION FOR SEQ ID NO:24:

(i) SEQUENCE CHARACTERISTICS:
(A) LENGTH: 792 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:24:

AGCTTTTAAC AAATTTCATT ATTTTCTGCT TGTTGGCCTT TCAATTTCTC ACCAAACGAT 60

GCATTCATAT GGTCAGTGGA CTGTTTCAGT TGTTCATTGG AATCTTGTAG CTGCTTAAGT 120

AGATTATCAC TTAAATCTCT TATCTTCTTC TCATGATTTT CCATAATCTG TTGTATGGCA 180

ATTTGTGTGG CATTCTGTAG TTTTTGGATT TCAATTTTCC AATCTTCATT CACTTTTGCT 240

ACATTTTGCG TTATACTATC AAATTCCAAT GATTGTTTGG CATGCAATTG ATCAAATTCT 300

GTTGAAATTT TTGAATTTGT TATCTTAATT GCTTCGCATT TTTGCTTGAG TGCTTGATCA 360

TATGTTTCCC GGAAATGATT GGGTTCACAT ATGTAACCCA TTTTAACATT ACAATCATTA 420

TCGTTCCATT GATATAAGGG CTTTGTATCC CATATATGGA CACAATGTTC TTGATGCTTA 480

TAATTATCGG GATTGTTTTC CGACCAGTAG GCAAAGGAGA ATGCTTGACC GGTGGGTGAC 540

CAGAAAAAGG GTCTGCCATA GTCACGACTT GAACTATACT CATCATTGCC GCCCAACCAT 600

AAATTATGAG ATTTTCCAAC GACCCGTTTC ACCAGATCGA TAATGGCATT ATTCTTATCA 660

GCACTTTCGA TTGTTACAAG TTGTTGATCA TGGCGGGCAC ATTCATGCCA GGCTTGATGC 720

CAATTGTACT TAAGTTCTGT TTCAATAAGA TATTCTCTGC CATCTAAAGC CTTTTGTAAT 780

TGGGGCACCA TG 792

(2) INFORMATION FOR SEQ ID NO:25:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:25:

GCTCGTATAA TGTGTGG 17

(2) INFORMATION FOR SEQ ID NO:26:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:26:

CCAGATCTGT TGTTGATCAT GGCGG 25

(2) INFORMATION FOR SEQ ID NO:27:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:27:

CCAGATCTTG CCATAGTCAC GACTT 25

(2) INFORMATION FOR SEQ ID NO:28:

(i) SEQUENCE CHARACTERISTICS:
(A) LENGTH: 24 base pairs
(B) TYPE: nucleic acid
(C) STRANDEDNESS: single

(D) TOPOLOGY: linear

(ii) MOLECULE TYPE: DNA (synthetic)

(xi) SEQUENCE DESCRIPTION: SEQ ID NO:28:

CCAGATCTTG AGTGCTTGAT CATA 24

(2) INFORMATION FOR SEQ ID NO:29:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:29:

CCAGATCTGC CAGGCTTGAT GCCAA 25

(2) INFORMATION FOR SEQ ID NO:30:

(i) SEQUENCE CHARACTERISTICS:
(A) LENGTH: 29 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:30:

His Ser Asp Ala Val Phe Thr Gly Asn Tyr Thr Lys Leu Arg Lys Gln
1               5                   10                  15

Leu Ala Ala Lys Lys Tyr Leu Asn Lys Ala Leu Lys Xaa
            20                  25

(2) INFORMATION FOR SEQ ID NO:31:

(i) SEQUENCE CHARACTERISTICS:
(A) LENGTH: 111 base pairs
(B) TYPE: nucleic acid

(C) STRANDEDNESS: double
(D) TOPOLOGY: linear

(ii) MOLECULE TYPE: DNA (synthetic)

(xi) SEQUENCE DESCRIPTION: SEQ ID NO:31:

GATCTTCATG CATTCTGATG CTGTATTCAC TGGTAACTAC ACTAAACTGC GTAAACAGCT 60

GGCTGCTAAG AAATACCTGA ACAAGGCTCT GAAGATGGGG ATCCTGTGAT A 111

(2) INFORMATION FOR SEQ ID NO:32:

(i) SEQUENCE CHARACTERISTICS:
(A) LENGTH: 111 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:32:

AGCTTATCAC AGGATCCCCA TCTTCAGAGC CTTGTTCAGG TATTTCTTAG CAGCCAGCTG 60

TTTACGCAGT TTAGTGTAGT TACCAGTGAA TACAGCATCA GAATGCATGA A 111

(2) INFORMATION FOR SEQ ID NO:33:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:33:

GATCTTCATG CATTCTGATG CTGTATT 27

(2) INFORMATION FOR SEQ ID NO:34:

(i) SEQUENCE CHARACTERISTICS:
(A) LENGTH: 28 base pairs

(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:34:

CACTGGTAAC TACACTAAAC TGCGTAAA 28

(2) INFORMATION FOR SEQ ID NO:35:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:35:

CAGCTGGCTG CTAAGAAATA CCTGAACA 28

(2) INFORMATION FOR SEQ ID NO:36:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:36:

AGGCTCTGAA GATGGGGATC CTGTGATA 28

(2) INFORMATION FOR SEQ ID NO:37:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:37:

CCAGTGAATA CAGCATCAGA ATGCATGAA 29

(2) INFORMATION FOR SEQ ID NO:38:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:38:

CCAGCTGTTT ACGCAGTTTA GTGTAGTTA 29

(2) INFORMATION FOR SEQ ID NO:39:
(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:39:

GAGCCTTGTT CAGGTATTTC TTAGCAG 27

(2) INFORMATION FOR SEQ ID NO:40:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:40:

AGCTTATCAC AGGATCCCCA TCTTCA 26

(2) INFORMATION FOR SEQ ID NO:41:

(i) SEQUENCE CHARACTERISTICS:
(A) LENGTH: 35 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:41:

Ile Phe Met His Ser Asp Ala Val Phe Thr Gly Asn Tyr Thr Lys Leu
1               5                   10                  15

Arg Lys Gln Leu Ala Ala Lys Lys Tyr Leu Asn Lys Ala Leu Lys Met
            20                  25                  30

Gly Ile Leu
        35